# EUROPEAN PATENT APPLICATION

(11) **EP 3 231 810 A1**
(43) Date of publication of application: **18.10.2017**
(21) Application number: 16165694.7
(22) Date of filing: 15.04.2016
(51) Int. Cl.: C07H 1/00, C07H 3/06

(54) **IMPROVED PREPARATION OF VACCINES AGAINST STREPTOCOCCUS PNEUMONIAE TYPE 3**

(71) Applicant: Max-Planck-Gesellschaft zur Förderung der Wissenschaften e.V., 80539 München (DE); Vaxxilon AG, 4153 Reinach (CH)
(72) Inventor: SEEBERGER, Peter H., 14532 Kleinmachnow (DE); PEREIRA, Claney Lebev, 12203 Berlin (DE); PARAMESWARAPPA, Sharavathi Guddehalli, 12203 Berlin (DE); CALOW, Adam Daniel James, 14197 Berlin (DE); MENOVA, Petra, 12489 Berlin (DE); BAEK, Ju Yuel, 14195 Berlin (DE)
(74) Representative: Arth, Hans-Lothar

(57) **Abstract**

The present invention relates to the preparation of a synthetic tetrasaccharide representing part of the repeating unit of the *Streptococcus pneumoniae* type 3 capsular polysaccharide as well as conjugates thereof. Said conjugates are particularly useful for prevention and/or treatment of diseases associated with *Streptococcus pneumoniae,* and more specifically of diseases associated with *Streptococcus pneumoniae* type 3. The disclosed synthetic method has the huge advantages over the state of the art synthetic methods that intermediate products are crystalline, coupling reaction yields are higher, less reaction steps are required and purification of intermediate products is easier.

## Description

### Specification

The present invention relates to the preparation of a synthetic tetrasaccharide representing part of the repeating unit of the *Streptococcus pneumoniae* type 3 capsular polysaccharide as well as conjugates thereof. Said conjugates are particularly useful for prevention and/or treatment of diseases associated with *Streptococcus pneumoniae,* and more specifically of diseases associated with *Streptococcus pneumoniae* type 3.

### Background of the invention

The Gram-positive bacterium *Streptococcus pneumoniae* is one of the main pathogens and causes severe invasive diseases like meningitis, pneumonia or bacteremia. Particularly infants, the elderly and immunocompromised patients are at high risk towards pneumococcal infections. About more than 90 serotypes of *Streptococcus pneumoniae* have been identified throughout the world, with a small number of these serotypes accounting for most diseases. The serotypes are identified by their different core capsular polysaccharide (CPS) structure. The CPS consists of polymers of repeating oligosaccharide units, which represent the epitope of the bacteria. These CPS or parts of them are often immunogenic and constitute potential candidates for pneumococcal vaccines.

The resistance of *Streptococcus pneumoniae* to antibiotics is a rapidly increasing problem. Thus, pneumococcal vaccines providing protection against pneumococcal infections are becoming increasingly important. Two classes of pneumococcal vaccines are currently available, one based on polysaccharides and the other based on polysaccharides conjugated to a carrier protein. The polysaccharides are thymus-independent type 2 antigens and thus induce low-affinity antibodies. In addition, they evoke no B-cell memory. Pneumococcal conjugate vaccines can circumvent these disadvantages with an increased serotype-specific antibody response to capsular polysaccharides. The conjugate vaccine PCV-13 contains immunogenic conjugates comprising the purified polysaccharides of 13 different S. *pneumoniae* serotypes covalently linked to a protein, such as CRM₁₉₇.

*Streptococcus pneumoniae* type 3 (ST3) is part of the current pneumococcal vaccines. The capsular polysaccharide of ST3 consists of [→3)-β-D-Glc*p*A-(1→4)-β-D-Glc*p*-(1→] repeating units. Immunization experiments with ST3 oligosaccharides conjugated to CRM₁₉₇ showed that increasing IgG antibody titers were found with increasing chain length (from monosaccharide to tetrasaccharide) and no influence on the immunogenicity by the oligosaccharide/CRM₁₉₇ ratio (see Benaissa-Trouw et al., Infection and Immunity, 2001, 69, 4698 - 4701).

Oligosaccharides derived from the repeating unit of S. *pneumoniae* type 3 can be obtained either by hydrolysis of the capsular polysaccharide of S. *pneumoniae* type 3 or synthetically. Hydrolysis of the capsular polysaccharide of S. *pneumoniae* type 3 results in a mixture of fragments of increasing degree of oligomerisation. Separation of the different fragments is difficult and due to practical considerations the conjugation chemistry available for polysaccharide-protein conjugation is basically restricted to reductive amination reactions (see for example: Snippe et al., Infection and Immunity, 1983, 42, 842 - 844). On the other hand, synthetic methods provide oligosaccharides with defined structures and the ability to use a much broader range of conjugation methods.

Lefeber et al. (Carbohydrate Research, 2002, 337, 819 - 825) teach the isolation of ST3 oligosaccharides with the repeating unit [→3)-β-D-Glc*p*A-(1→4)-β-D-Glc*p-*(1→]ₙ, wherein n = 1 - 7. The ST3 oligosaccharides are isolated from a partial-acid hydrolysate of the capsular polysaccharide of S. *pneumoniae* type 3 by Sepharose Q ion-exchange chromatography. The isolated fragments were contaminated by side-products originating from cleavage of β-D-Glc*p*A-(1→4)-β-D-Glcp linkage.

Lefeber et al. (Chemistry - Eur. J. 2001, 7, 4411 - 4421) also disclose the synthesis of a tetrasaccharide derived from the repeating unit of S. *pneumoniae* type 3 linked to 3-aminopropanol and the synthesis of a hexasaccharide derived from the repeating unit of S. *pneumoniae* type 3 (Canadian Journal of Chemistry 2002, 80, 76 - 81). The approach consists of sequentially coupling 4,6-benzylidene protected glucose molecules with 6-benzoyl protected glucose to di-, tri- or tetrasaccharide. Subsequent selective removal of benzylidene groups and selective oxidation of primary 6-OH group introduces the carboxylic groups. Glycosylation is realized by trichloroacetimidate activated glucosyl donors and O-allyl protected glucosyl acceptors. The 3-aminopropanol spacer is introduced as 3-azido-1-propanol and the azide group reduced to amino group during the deprotection of the oligosaccharide. The 3-aminopropyl glycoside is conjugated to a carrier protein (i.e. CRM₁₉₇, KLH or TT) using the squarate coupling method. The approach makes this synthesis elaborate for longer oligosaccharides, because only one glucose unit can be introduced in each step.

Rathwell et al. (EP 2 851 092) disclose protein- and peptide-free synthetic vaccines against *S*. *pneumoniae* type 3. The oligosaccharides derived from the repeating unit of *S*. *pneumoniae* type 3 are synthesized in a sequential manner, i.e. repeatedly coupling of suitably protected glucose disaccharide fragments to spacer-containing fragment and subsequent introduction of carboxylic acid groups by selective oxidation of the 4,6-deprotected glucose parts. The amine functionalized oligosaccharide is conjugated to a glycosphingolipid (GSL) derived from galactosyl ceramide. The Synthesis of the oligosaccharide proceeded only in moderate yields due to the TBS group (tert-butyldimethylsilyl) at position 3 of the glucuronic acid moiety. Partial cleavage of the TBS group was observed under these reaction conditions. While elongation of the carbohydrate is achieved with a disaccharide fragment, the synthesis of longer oligosaccharides is still elaborate.

Thus, the state of the art discloses the synthesis of oligosaccharides derived from the repeating unit of *S*. *pneumoniae* type 3 as well as their conjugation to proteins and glycosphingolipids

However these syntheses are elaborate, contain many tedious reaction steps with low or moderate yields and are therefore not practical for the synthesis of oligosaccharides derived from the repeating unit of *S*. *pneumoniae* type 3 with more than 4 repeating units. Thus a convenient synthesis is so far not known in the state of the art.

It is the objective of the present invention to provide a method for the synthesis of a tetrasaccharide derived from the repeating unit of S. *pneumoniae* type 3 suitable for covalently binding to carrier proteins like CRM197 or TT or to a glycosphingolipid. Pharmaceutical compositions of said conjugates are useful for prevention and/or treatment of diseases associated with *Streptococcus pneumoniae,* and more specifically of diseases associated with *Streptococcus pneumoniae* type 3. Immunization with said conjugates results in the production of high titers of antibodies against pneumococcal capsular polysaccharide of ST3. The antibodies present opsonophagocytosis activity and bactericidal activity.

The objective of the present invention is solved by the teaching of the independent claims. Further advantageous features, aspects and details of the invention are evident from the dependent claims, the description, the figures, and the examples of the present application.

### Brief description of the invention

The present invention relates to a synthetic method for preparation of tetrasaccharide derived from the repeating unit of *S*. *pneumoniae* type 3 of formula **1,** 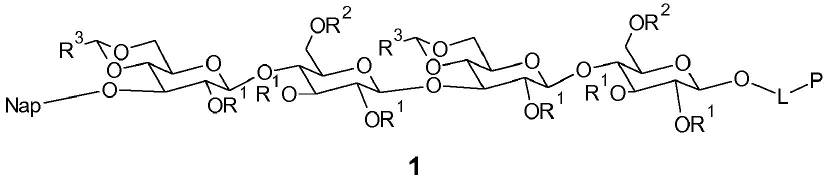 wherein R¹ and R² represent protecting groups, R³ is an aryl or alkyl group and -O-L-P is a protected linker for conjugation to a carrier protein or glycosphingolipid.

Formation of the tetrasaccharide of formula 1 is achieved by coupling the two disaccharides of formula **2** and **3** in the presence of a catalyst, 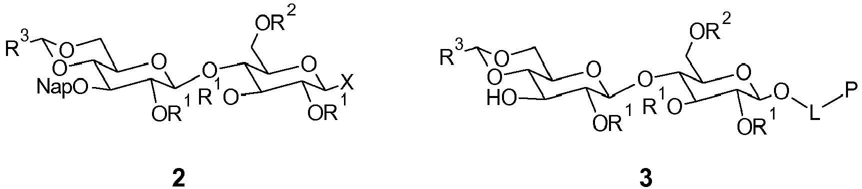 wherein X is a leaving group and -O-L-P is a protected linker for conjugation to a carrier protein or glycosphingolipid. The coupling of the disaccharides of formula **2** and **3** is a key step of the synthesis of longer saccharides such as tetrasaccharide **1.**

One aspect of the innovation of this improved synthesis relies on the use of the 2-naphthylmethyl protecting group (Nap). The Nap group can be easily installed and selectively removed under mild conditions, i.e. in the presence of DDQ (2,3-dichloro-5,6-dicyano-1,4-benzoquinone) or ceric ammonium nitrate (CAN) or by hydrogenation. Despite the facile removal under mild conditions, Nap-containing intermediates are stable under coupling reaction conditions. No side reactions occurred during synthesis due to undesired removal of Nap group. Other protecting groups like TBS-ether showed partial cleavage under coupling reaction conditions. Therefore, increased yields are achieved by using the Nap group, especially in the formation of the tetrasaccharide of formula **1.**

Surprisingly, it was found that all Nap-protected products and intermediates were crystalline. This facilitated the purification procedures during synthesis. All Nap-protected products and intermediates could be purified by recrystallization or by trituration. The use of time- and material-consuming column chromatography for purification was avoided in many steps. Thus, the use of the Nap group facilitates performing the synthesis and further allows scaling up the synthesis without significant increase of required time and material.

Another aspect of the present invention is that the tetrasaccharide of formula **1** contains a protected linker that following deprotection enables conjugation to a protein or glycosphingolipid, at the reducing end.

A further aspect of the disclosed invention relates to the deprotection of the tetrasaccharide of formula **1** bearing a protected linker. Removal of the acetal groups under addition of an acid is followed by selective oxidation of the primary 6-OH-group. Subsequent removal of the remaining protective groups and simultaneous deprotection of the linker lead to the deprotected tetrasaccharide of formula **8,** 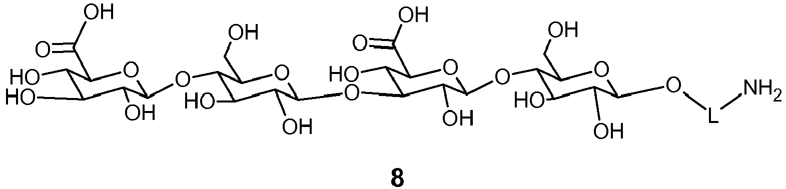 wherein L may be an aliphatic or aromatic residue, e.g. an alkyl(en) group or phenyl(en) group.

In a further aspect, the unprotected tetrasaccharide of formula **8** bearing a reactive linker is conjugated to a carrier protein or a glycosphingolipid. The carrier protein can be any carrier protein known in the art, in particular in the field of vaccine development. More specifically, the carrier protein is selected from the group comprising diphtheria toxoid CRM₁₉₇, tetanus toxoid (TT), outer membrane protein (OMP), bovine serum albumin, (BSA), keyhole limpet hemocyanine (KLH), diphtheria toxoid (DT), cholera toxoid (CT), recombinant *Pseudomonas aeruginosa* exotoxin A (rEPA), Clostridium difficile toxin A (TcdA), Clostridium difficile toxin B (TcdB).

Thus the synthetic method disclosed herein has huge unexpected advantages over the state of the art synthetic methods that intermediate products are crystalline, coupling reaction yields are higher, less reaction steps are required and purification of intermediate products is easier. Therefore, the improved method is less time and material consuming, less costly and easier to scale up because column chromatography is in many reaction steps no longer necessary.

### Detailed Description of the invention

### Definitions

The linker or spacer group herein disclosed may be any moiety that enables to couple the oligosaccharide to a carrier molecule, i.e. a carrier protein or a glycosphingolipid. A large variety of such linker groups are known in the art and a suitable linker group can be selected in dependence from the respective carrier molecule. For example, the linker may be an aliphatic or aromatic residue, e.g. an alkyl(en) group or phenyl(en) group, comprising a reactive functional group, such as an amino group, preferably a primary amino group, (activated) carboxy group, aldehyde, azide, alkenyl or alkynyl group. In specific embodiments the linker may comprise a polyether or polyester chain. In particular, the linker is selected from the group comprising primary alkylamines, alkyl or aralkyl residues with a terminal aldehyde, azide, alkyne or alkene group or (activated) carboxy group, and alkylaryl and aryl residues, e.g. phenyl residues, comprising a reactive amine, an aldehyde or an azide group, or (activated) carboxy group.

In a specific embodiment of the invention, the linker is -(CH₂)ₙ-NH₂, with n being an integer from 2 to 10, preferably 2 to 8, more preferably 2 to 5.

The invention concerns a synthetic method for preparation of a ST3 tetrasaccharide bearing a linker.

The present invention provides very favorable and efficient methods for synthesizing the ST3 tetrasaccharide and ST3 tetrasaccharide conjugates selectively and in high yields.

### Retrosynthetic analysis

The inventive method for preparation of the tetrasaccharide of formula **8** is outlined in Scheme 1 below. The synthetic method comprises the following steps:
- construction of monosaccharide building block of formula **10** from thioglucoside of formula **14**
- coupling monosaccharide building blocks of formula **10** and **11** or **11** and **12** to disaccharide of formula **2** with special consideration being given to assure that only the β-anomer is formed,
- converting disaccharide of formula **2** to disaccharide of formula **3,** which comprises coupling disaccharide of formula **2** with a protected linker and subsequent selective removal of the Nap-group,
- coupling disaccharides of formula **2** and **3** to fully protected tetrasaccharide of formula **1** bearing a protected linker at the reducing end with special consideration being given to assure that only the β-anomer is formed,
- selective removal of the acetal groups of fully protected tetrasaccharide of formula **1,**
- introduction of carboxylic acid groups by selective oxidation of the primary 6-OH-groups of protected tetrasaccharide of formula **4',**
- removal of the remaining protecting groups, i.e. Nap, R¹, R² and protecting groups of the linker to obtain deprotected tetrasaccharide of formula **8.**

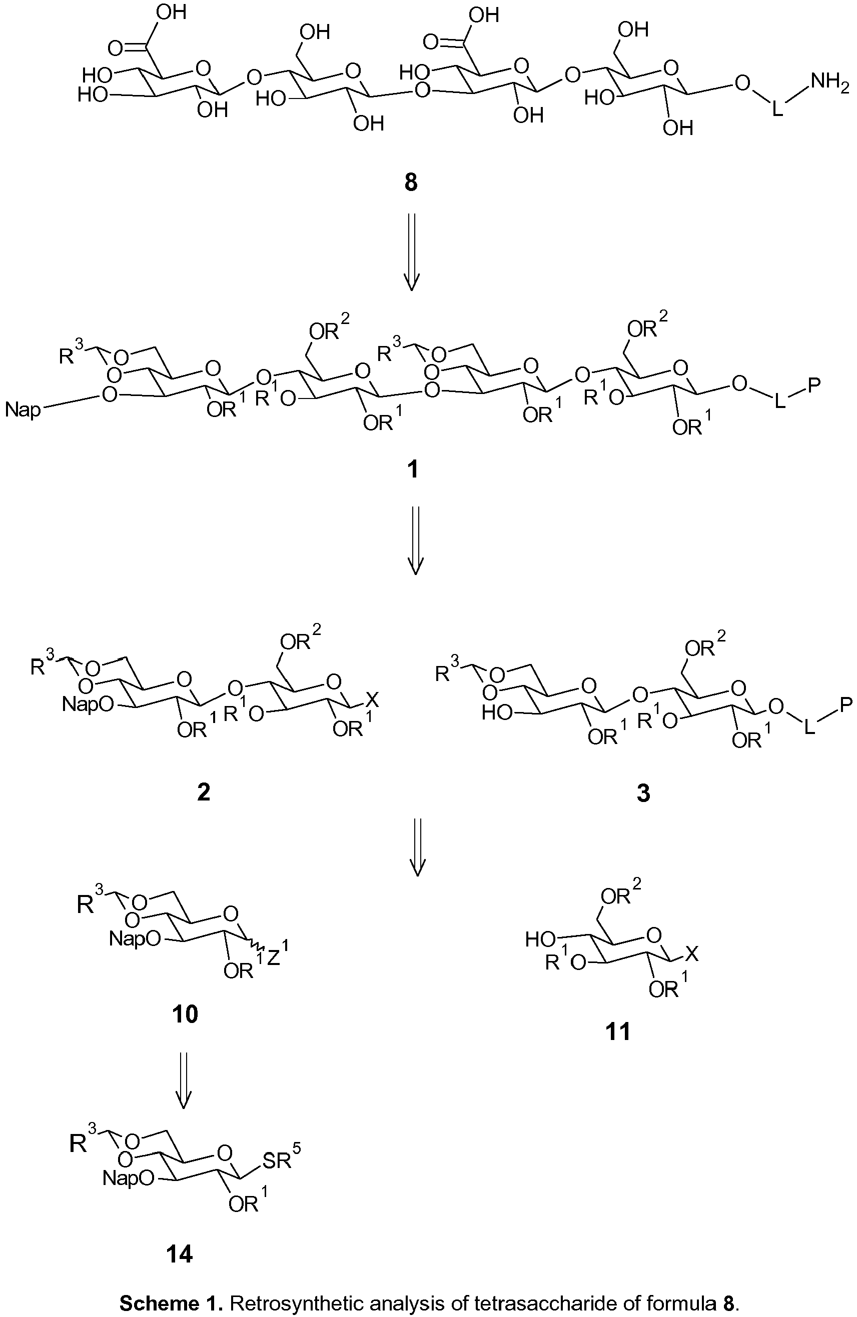

The method follows the approach of first constructing the oligosaccharide containing only glucose fragments and subsequent oxidizing every second glucose fragment to glucuronic acid fragment.

### Protecting group strategy

It is necessary for the tetrasaccharide synthesis to provide a set of orthogonal protecting groups. In order to construct the tetrasaccharide of formula **8** from disaccharides of formula **2,** the ability to selectively and independently remove the Nap-group and the protecting group R³ in the presence of all other protecting groups is essential. The term "protecting group" or "protective group" as used herein refers to commonly used groups in organic synthesis, preferably used for protection of amines, hydroxyl groups, thiols, imines, carbonyls, carboxyls or other common functional groups, and particularly preferred for amines and hydroxyl groups. The protecting groups are characterized in that they are stable under reaction conditions applied during the synthesis, i.e. they are not cleaved off or undergo undesired side reactions and prevent any reaction of the protected functional group they are bonded to. Additionally, the protecting groups are selected to not hinder or to not affect the performed reaction steps in terms of yield or stereoselectivity.

The protecting groups R¹ and R² are present during the synthesis and removed in the last deprotection step(s). R¹ and R² are therefore regarded as permanent protecting groups. On the other hand, the Nap-group and R³ are removed during the synthesis and are therefore temporary protecting groups.

In order to selectively and independently remove the Nap-group in the presence of the permanent protecting groups R¹ and R², the permanent protecting groups have to be stable towards an oxidizing agent, i.e. DDQ and CAN. As used herein the term "oxidation-stable protecting group" refers to a protecting group that is stable during oxidation reaction performed with DDQ or CAN as reagents. Thus, the permanent protecting groups R¹ and R² have to be oxidation-stable. In addition the permanent protecting groups R¹ and R² have to be stable under acidic conditions, which are applied to remove the acetal protecting group installed at the 4^{th} and 6^{th} position of the glucose moiety of the tetrasaccharide **1** and which are also applied in the glycosylation reactions. The acetal protecting group installed at the 4^{th} and 6^{th} position of the glucose moiety of the tetrasaccharide **1** can be removed by treatment with a nucleophile in presence of a catalytic amount of an acid. Thus, protecting groups R¹ and R² have to be stable upon treatment of the saccharide with a nucleophile and a catalytic amount of acid. Examples of nucleophiles that can be used in the removal of the acetal protecting group installed at the 4^{th} and 6^{th} position of the glucose moiety of the tetrasaccharide **1** include, but are not restricted to: water, alcohol and thiol. Preferably, the nucleophile is a thiol, such as ethanethiol (EtSH), tolylthiol, phenylthiol, ethylenethiol and more preferably the nucleophile is ethanethiol (EtSH). Examples of acids that can be used in catalytic amount for the removal of the acetal protecting group installed at the 4^{th} and 6^{th} position of the glucose moiety of the tetrasaccharide **1,** include but are not restricted to the following Bronstedt acids: methanesulfonic acid, p-toluenesulfonic acid, camphorsulfonic acid (CSA) and trifluoromethanesulfonic acid and the Lewis acid Er(OTf)₃.

Glycosylation reactions take place upon treatment of a donor and an acceptor with an activator or an activating agent: Activators or activating agents known to the skilled person include, but are not restricted to: AgOTf, BF₃•OEt₂, trimethylsilyl trifluoromethanesulfonate (TMSOTf), trifluoromethanesulfonic acid (TfOH), trifluoromethanesulfonic anhydride (Tf₂O, triflic anhydride), lanthanoid(III) triflates, NIS/AgOTf, NIS/TfOH or dimethyl(methylthio)sulfonium trifluoromethanesulfonate (DMTST). Preferably the activator or activating agent is NIS/TfOH.
Hence, permanent protecting groups R¹ and R² have to be stable under treatment of the saccharides with any of the above-mentioned activating agents.

As used herein the term "acid-stable protecting group" refers to a protecting group which is stable in the presence of a nucleophile and catalytic amounts of an acid as well as in the presence of an activating agent.

Preferably, acid-stable protecting groups R¹ and R² are protecting groups which are stable in the presence of ethanethiol and p-toluenesulfonic acid as well as in the presence of NIS/TfOH.

Consequently, the permanent protecting groups R¹ and R² have to be oxidation-stable i.e. have to be stable upon treatment with the oxidizing agent required for removal of the Nap protecting group (e.g. DDQ or CAN) and acid-stabile.

The protecting group R¹, which also blocks the 2-OH-group, is chosen to facilitate the formation of β-anomers in glycosylation reactions, because the ST3 repeating unit consists only of β-anomers of glucose and glucuronic acid. The formation of β-anomers can be significantly promoted when the protecting group employed at the 2-OH-group of the glucosyl donor participates in the glycosylation reaction so that it blocks the lower side of the anomeric center. Therefore the β-anomer is predominantly formed. Such participating groups are known from the state of the art. Thus, the protecting group R¹ is preferably an ester group, more preferably a benzoyl, chloroacetyl (ClAc), acetyl or pivaloyl (Piv) group and even more preferably a benzoyl group.

For the protecting group R² the same limitations hold true, i.e. oxidation-stable and acid-stabile. However, R² does not need to participate in the coupling reaction. R² may be an ester group, a benzyl group or a TBS-group. More preferably, R² is a benzyl group.

The acetal protecting group R³ may be any alkyl or aryl group. Preferred are phenyl and substituted phenyl groups like 4-methoxyphenyl, 4-nitrophenyl and 2-nitrophenyl. Even more preferred is when R³ represents a phenyl group.

Accordingly, the functional group of the linker has to be protected with oxidation-stable and acid-stable protecting group(s) during the ST3 tetrasaccharide synthesis. Particularly, when the functional group is a primary amine, it can be protected preferably but not exclusively as: -N(Bn)Cbz, -NHCbz, -N₃, -NBn₂, -NAllyl₂ or -NPhth. When the protected functional group can be written as -NR'R", -NPhth can be understood as R' and R" form together with the nitrogen atom they are attached to a phthalimide or R and R form together phthaloyl. Methods for deprotection or conversion of such groups into primary amines are known to the skilled person.

The term "coupling reaction" herein used refers to reactions between two saccharides wherein one reducing end reacts or the term refers to reactions between one saccharide and an alcohol. Consequently, O-glycosylation methods are employed in the coupling reaction steps of the synthetic method according to the invention. These O-glycosylation methods are known from the state of the art. Generally, they require a leaving group at the reducing end of the donor, which is activated in the presence of a catalyst. Leaving groups can be selected in dependence on the used catalyst and may be for example: a halogen, -O-C(=NH)-CCl₃, -O-C(=NPh)-CF₃, -OAc, -SR⁵, -SO-Ph, -O-(CH₂)₃-CH=CH₂, -O-P(OR⁵)₂, -O-PO(OR⁵)₂, -O-CO-OR⁵, -O-CO-SR⁵, -O-CS-SR⁵, -O-CS-OR⁵, 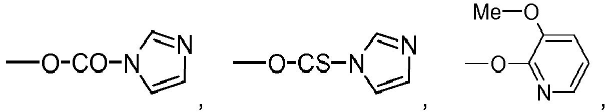 wherein R⁵ may be any alkyl or aryl group.

In a coupling or glycoslation reaction any suitable catalyst or promoter or activator or combinations thereof for O-glycosylation reactions are known in the art, i.e. Brønstedt acids or Lewis acids, may be employed. Specifically, these acids can be selected from the non-exhaustive list comprising AgOTf, BF₃•OEt₂, trimethylsilyl trifluoromethanesulfonate (TMSOTf), trifluoromethanesulfonic acid (TfOH), trifluoromethanesulfonic anhydride (Tf₂O, triflic anhydride), lanthanoid(III) triflates, NIS/AgOTf, NIS/TfOH or dimethyl(methylthio)sulfonium trifluoromethanesulfonate (DMTST).

### Construction of monosaccharide building blocks 10 and 11

The monosaccharide building block **10** is prepared from thioglucoside **14.** The preparation of thioglucoside **14** is known to a skilled person, especially when R¹ = -Bz, R³ = -Ph and R⁵ = -Et. Thioglucoside **14** can be formed starting from glucose diacetonide **17** (see Scheme 2) or is also commercially available, when R¹ = -Bz, R³ = -Ph and R⁵ = -Et. The residue R⁵ of the thioglucosides **14, 21, 22** and **23** may contain any alkyl or aryl group. Preferably, R⁵ is either an ethyl group or a tolyl group.

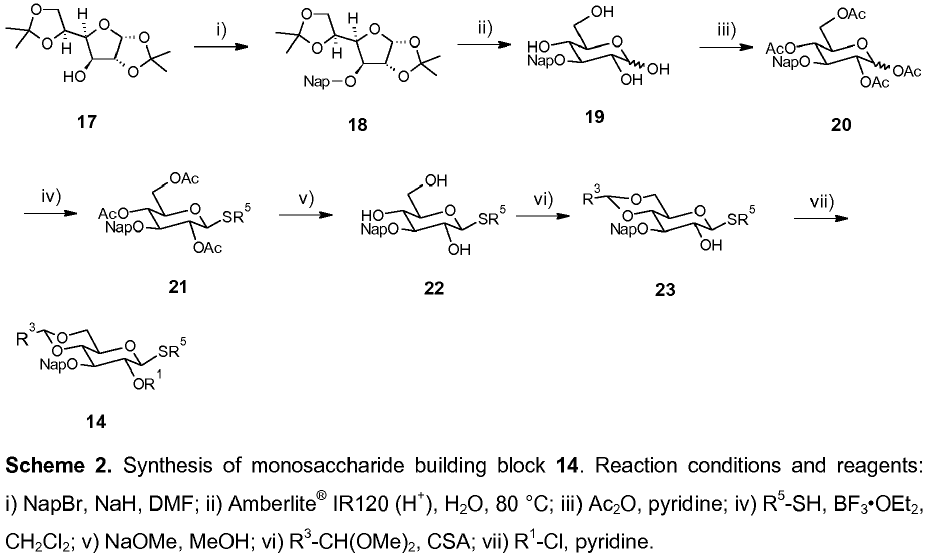

Starting from glucose diacetonide **17** the 3-OH group is blocked by 2-naphthylmethyl bromide in the presence of sodium hydride. Subsequent removal of acetonide groups of glucose derivative **18** leads to 3-Nap-protected glucose **19** which is then completely acetylated. Peracetylated glucose **20** is then converted to thioglucoside **21.** Deacetylation is achieved in the presence of a base and by acetalization with an aldehyde or preferably the corresponding dialkyl acetal and a strong acid the 4-OH and 6-OH groups are selectively blocked. Finally, the remaining 2-OH-group of **22** is protected as an ester.

### Preparation of monosaccharide of formula 10

An aspect of the present invention is directed to a method of synthesis of the monosaccharide of formula **10** from monosaccharide building block of formula **14** as outlined in Scheme 3 below.

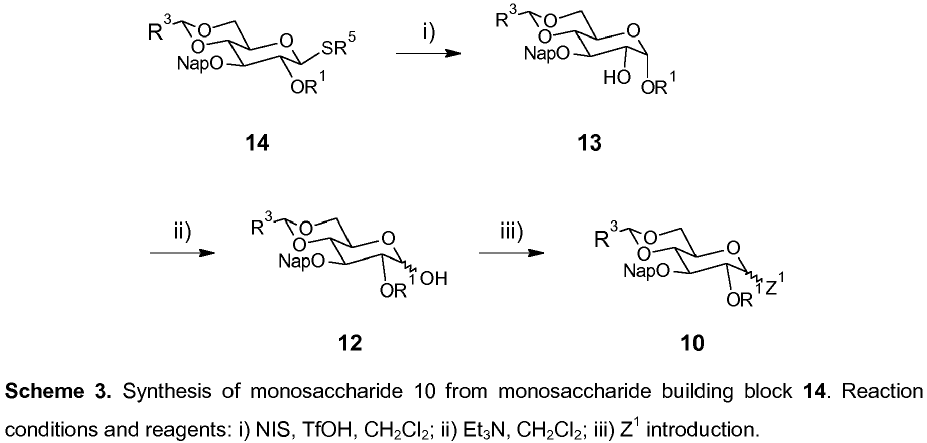

The inventive method comprises the following steps:
a3) Providing a monosaccharide of formula **14** 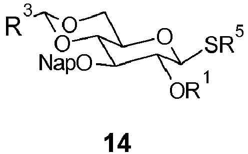 wherein
   R¹ is an oxidation-stable and acid-stable protecting group,
   R³ is an aryl or alkyl group,
   R⁵ is selected from -Me, -Et, -Pr, -Bu, -Ph, and -Tol;
b3) Converting the monosaccharide of formula **14** to a monosaccharide of formula **13** 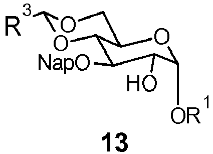 wherein
   R¹ is an oxidation-stable and acid-stable protecting group,
   R³ is an aryl or alkyl group; and
c3) Treatment of the monosaccharide of formula **13** with a base to obtain a monosaccharide of formula **12** 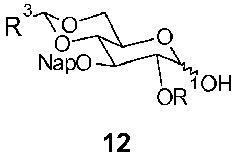 wherein
   R¹ is an oxidation-stable and acid-stable protecting group,
   R³ is an aryl or alkyl group;
      and
d3) converting the monosaccharide of formula **12** to the monosaccharide of formula **10** 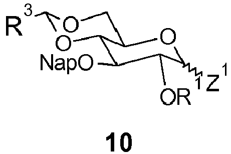 wherein
   R¹ represents an oxidation-stable and acid-stable protecting group,
   R³ is an aryl or alkyl group
   and Z¹ is a leaving group.

It is preferred that the method comprises the following steps:
a3) Providing a monosaccharide of formula **14** wherein
   R¹ is selected from -Bz, -ClAc, -Ac and -Piv, R³ is selected from -Me, -Et, -Ph, -tBu and -PMP, and R⁵ is selected from -Me, -Et, -Pr, -Bu, -Ph and -Tol;
b3) Converting the monosaccharide of formula **14** to a monosaccharide of formula **13;** and c3) Treatment of the monosaccharide of formula **13** with a base to obtain a monosaccharide of formula **12;** and d3) converting the monosaccharide of formula **12** to the monosaccharide of formula **10,** wherein Z¹ is selected from -O-C(=NH)-CCl₃, -O-C(=NPh)-CF₃, -OAc and -SR⁵.

More preferably, the method according to the invention comprises the following steps: a3) Providing a monosaccharide of formula **14** wherein R¹ represents -Bz, R³ is selected from -Me, -Et, -Ph, -tBu and -PMP, and R⁵ is selected from - Me, -Et, -Pr, -Bu, -Ph, and -Tol;
b3) Converting the monosaccharide of formula **14** to a monosaccharide of formula **13;** and c3) treatment of the monosaccharide of formula **13** with a base to obtain a monosaccharide of formula **12;** and; d3) converting the monosaccharide of formula **12** to the monosaccharide of formula **10,** wherein Z¹ is selected from -O-C(=NH)-CCl₃, -O-C(=NPh)-CF₃, -OAc and -SR⁵.

More preferably, the method according to the invention comprises the following steps: a3) Providing a monosaccharide of formula **14** wherein R¹ is selected from -Bz, -ClAc, -Ac and -Piv, R³ represents, -Ph, R⁵ is selected from -Me, -Et, -Pr, -Bu, -Ph,and -Tol;
b3) Converting the monosaccharide of formula **14** to a monosaccharide of formula **13;** and c3) treatment of the monosaccharide of formula **13** with a base to obtain a monosaccharide of formula **12;** and d3) converting the monosaccharide of formula **12** to the monosaccharide of formula **10,** wherein Z¹ is selected from -O-C(=NH)-CCl₃, -O-C(=NPh)-CF₃, -OAc or -SR⁵.

Even more preferably, the method according to the invention comprises the following steps: a3) Providing a monosaccharide of formula **14** wherein R¹ is selected from -Bz, -ClAc, -Ac and -Piv, R³ is selected from -Me, -Et, -Ph, -tBu and -PMP, R⁵ represents -Et;
b3) Converting the monosaccharide of formula **14** to monosaccharide of formula **13;** and c3) treatment of the monosaccharide of formula **13** with a base to obtain a monosaccharide of formula **12;** and d3) converting the monosaccharide of formula **12** to the monosaccharide of formula **10,** wherein Z¹ is selected from -O-C(=NH)-CCl₃, -O-C(=NPh)-CF₃, -OAc and -SR⁵, wherein R⁵ is selected from -Me, -Et, -Pr, -Bu, -Ph, and -Tol.

Even more preferably, the method according to the invention comprises the following steps: a3) Providing a monosaccharide of formula **14** wherein R¹ is selected from -Bz, -ClAc, -Ac and -Piv, R³ is selected from -Me, -Et, -Ph, -tBu and -PMP, R⁵ is selected from -Me, -Et, -Pr, -Bu, -Ph, and -Tol;
b3) Converting the monosaccharide of formula **14** to a monosaccharide of formula **13;** and c3) treatment of the monosaccharide of formula **13** with a base to obtain a monosaccharide of formula **12;** and d3) converting the monosaccharide of formula **12** to the monosaccharide of formula **10,** wherein Z¹ represents -O-C(=NPh)-CF₃. Even more preferably, the method according to the invention comprises the following steps: a3) Providing a monosaccharide of formula **14** wherein R¹ is selected from -Bz, and -Ac, R³ represents -Ph, R⁵ represents -Et;
b3) Converting the monosaccharide of formula **14** to a monosaccharide of formula **13;** and c3) treatment of the monosaccharide of formula **13** with a base to obtain a monosaccharide of formula **12;** and d3) converting the monosaccharide of formula **12** to the monosaccharide of formula **10,** wherein Z¹ represents -O-C(=NPh)-CF₃.

It is preferred that the conversion of monosaccharide of formula **14** to monosaccharide of formula **13** is performed in the presence of NIS and catalytic amounts of a strong acid in a mixture of water and an aprotic apolar solvent at low temperatures between -20°C and 10°C. Even more preferred is that the conversion of monosaccharide of formula **14** to monosaccharide of formula **13** is performed in the presence of NIS and catalytic amounts of trifluoromethanesulfonic acid in a mixture of water and CH₂Cl₂ at 0°C.

It is also preferred that the reaction of monosaccharide of formula **13** to monosaccharide of formula **12** is performed in the presence of a base in an aprotic apolar solvent. Even more preferred is that the reaction of monosaccharide of formula **13** to monosaccharide of formula **12** is performed in the presence of triethylamine in CH₂Cl₂.

It is preferred that the conversion of monosaccharide of formula **12** to monosaccharide of formula **10** is achieved by an imidoyl chloride, or trihalogenated acetonitrile in the presence of a base in an aprotic apolar solvent. More preferred is that the conversion of monosaccharide of formula **12** to monosaccharide of formula **10** is achieved by 2,2,2-trifluoro-N-phenylacetimidoyl chloride in the presence of Cs₂CO₃ and/or K₂CO₃ in methylene chloride. According to the present invention the monosaccharide of formula **10** is preferably obtained in form of crystals by recrystallization or trituration.

Thus, it is especially preferred that the method for the preparation of monosaccharide of formula **10** comprises the following steps: a3) Providing a monosaccharide of formula **14** with R¹ representing benzoyl, R³ is a phenyl group and R⁵ representing ethyl; b3) Converting the monosaccharide of formula **14** to a monosaccharide of formula **13;** and c3) treatment of the monosaccharide of formula **13** with triethylamine to obtain a monosaccharide of formula **12;** and d3) converting the monosaccharide of formula **12** to the monosaccharide of formula **10**with Z¹ representing -O-C(=NPh)-CF₃.

### Preparation of disaccharide of formula 2

A further aspect of the present invention relates to a method for preparation of disaccharide of formula 2, which comprises the following steps:
a2) Providing a monosaccharide building block of formula **10** 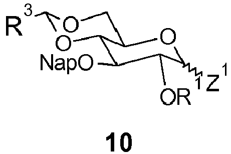 wherein
   R¹ represents an oxidation-stable and acid-stable protecting group,
   R³ is an aryl or alkyl group
   and Z¹ is a leaving group,
      and a monosaccharide building block 11 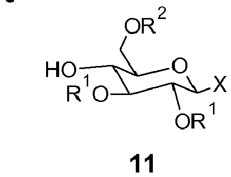 wherein

   R¹ represents an oxidation-stable and acid-stable protecting group,
   R² represents an oxidation-stable and acid-stable protecting group, and
   X is a leaving group;
      and
b2) Coupling of the monosaccharide building block of formula **10** with the monosaccharide building block of formula **11** in presence of a catalyst to yield disaccharide of formula **2** 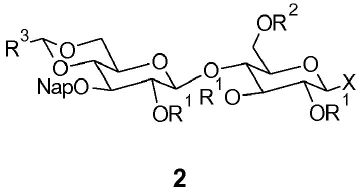 wherein
   R¹ represents an oxidation-stable and acid-stable protecting group,
   R² represents an oxidation-stable and acid-stable protecting group,
   R³ is an aryl or alkyl group,
   X is a leaving group.

It is preferred that the method for the preparation of disaccharide of formula 2 comprises the following steps:
a2) Providing a monosaccharide building block of formula 10 wherein R¹ is selected from -Bz, -ClAc, -Ac and -Piv, R³ is selected from -Me, -Et, -Ph, -tBu and -PMP and Z¹ is selected from -O-C(=NH)-CCl₃, -O-C(=NPh)-CF₃, -OAc and -SR⁵ with R⁵ is selected from -Me, -Et, -Pr, -Bu, -Ph, and -Tol and a monosaccharide building block 11 wherein R² is selected from -Bz, -Ac, -Piv, -Bn and -TBS, X represents -SR⁵ and R⁵ is selected from -Me, -Et, -Pr, -Bu, - Ph, and -Tol;
b2) Coupling of the monosaccharide building block of formula 10 with the monosaccharide building block of formula **11** in presence of a catalyst to yield disaccharide of formula **2.**

It is also preferred that the method for the preparation of disaccharide of formula **2** comprises the following steps:
a2) Providing a monosaccharide building block of formula **10** wherein R¹ represents -Bz, R³ is selected from -Me, -Et, -Ph, -tBu and -PMP and Z¹ is selected from -O-C(=NH)-CCl₃, -O-C(=NPh)-CF₃, -OAc and -SR⁵ with R⁵ is selected from -Me, -Et, -Pr, -Bu, -Ph, and -Tol and a monosaccharide building block **11** wherein R² is selected from -Bz, -Ac, -Piv, -Bn and -TBS, X represents -SR⁵ and R⁵ is selected from -Me, -Et, -Pr, -Bu, -Ph, and -Tol;
b2) Coupling of the monosaccharide building block of formula **10** with the mono-saccharide building block of formula **11** in presence of a catalyst to yield disaccharide of formula **2.**

It is even more preferred that the method for the preparation of disaccharide of formula **2** comprises the following steps:
a2) Providing a monosaccharide building block of formula **10** wherein R¹ is selected from -Bz, -ClAc, -Ac and -Piv, R³ represents -Ph and Z¹ is selected from -O-C(=NH)-CCl₃, -O-C(=NPh)-CF₃, -OAc and -SR⁵ with R⁵ is selected from -Me, -Et, -Pr, -Bu, -Ph, and -Tol and a monosaccharide building block **11** wherein R² is selected from -Bz, -Ac, -Piv, -Bn and -TBS, X represents -SR⁵ and R⁵ is selected from -Me, -Et, -Pr, -Bu, -Ph, and -Tol;
b2) Coupling of the monosaccharide building block of formula **10** with the monosaccharide building block of formula **11** in presence of a catalyst to yield disaccharide of formula **2.**

It is even more preferred that the method for the preparation of disaccharide of formula **2** comprises the following steps:
a2) Providing a monosaccharide building block of formula **10** wherein R¹ is selected from -Bz, -ClAc, -Ac and -Piv, R³ is selected from -Me, -Et, -Ph, -tBu and -PMP and Z¹ is selected from -O-C(=NH)-CCl₃, -O-C(=NPh)-CF₃, -OAc and -SR⁵ with R⁵ is selected from -Me, -Et, -Pr, -Bu, -Ph, and -Tol and a mono-saccharide building block **11** wherein R² represents -Bn, X represents -SR⁵ and R⁵ is selected from -Me, -Et, -Pr, -Bu, -Ph, and -Tol;
b2) Coupling of the monosaccharide building block of formula **10** with the mono-saccharide building block of formula **11** in presence of a catalyst to yield disaccharide of formula **2.**

It is even more preferred that the method for the preparation of disaccharide of formula **2** comprises the following steps:
a2) Providing a monosaccharide building block of formula **10** wherein R¹ is selected from -Bz, -ClAc, -Ac and -Piv, R³ is selected from -Me, -Et, -Ph, -tBu and -PMP and Z¹ is selected from -O-C(=NH)-CCl₃, -O-C(=NPh)-CF₃, -OAc and -SR⁵ with R⁵ is selected from -Me, -Et, -Pr, -Bu, -Ph, and -Tol and a mono-saccharide building block **11** wherein R² is selected from -Bz, -Ac, -Piv, -Bn and -TBS, X represents -SEt;
b2) Coupling of the monosaccharide building block of formula **10** with the mono-saccharide building block of formula **11** in presence of a catalyst to yield disaccharide of formula **2.**

It is even more preferred that the method for the preparation of disaccharide of formula **2** comprises the following steps:
a2) Providing a monosaccharide building block of formula **10** wherein R¹ is selected from -Bz, -ClAc, -Ac and -Piv, R³ is selected from -Me, -Et, -Ph, -tBu and -PMP and Z¹ represents -O-C(=NPh)-CF₃ and a monosaccharide building block **11** wherein R² is selected from -Bz, -Ac, -Piv, -Bn and -TBS, X represents-SR⁵ and R⁵ is selected from -Me, -Et, -Pr, -Bu, -Ph, and -Tol;
b2) Coupling of the monosaccharide building block of formula **10** with the mono-saccharide building block of formula **11** in presence of a catalyst to yield disaccharide of formula **2**

It is even more preferred that the method for the preparation of disaccharide of formula 2 comprises the following steps:
a2) Providing a monosaccharide building block of formula **10** wherein R¹ is selected from -Bz, and -Ac, R³ represents -Ph, and Z¹ represents -O-C(=NPh)-CF₃ and a monosaccharide building block **11** wherein R² represents -Bn and X is -SEt; b2) Coupling of the monosaccharide building block of formula **10** with the monosaccharide building block of formula **11** in presence of a catalyst to yield disaccharide of formula **2.**

Preferably, the coupling of the monosaccharide building block of formula **10** with the monosaccharide building block of formula **11** is performed in the presence of catalytic amounts of a Lewis acid in an aprotic apolar solvent at temperatures between -78 °C and -20 °C. More preferably, the coupling of monosaccharide building block of formula **10** with monosaccharide building block of formula **11** is performed in the presence of catalytic amounts of TMSOTf or TfOH in an aprotic apolar solvent at temperatures between -78 °C and -20 °C. More preferably, the coupling of monosaccharide building block of formula **10** with monosaccharide building block of formula **11** is performed in the presence of catalytic amounts of TMSOTf or TfOH in methylene chloride or toluene at temperatures between -78 °C and -20 °C. More preferably, the coupling of monosaccharide building block of formula **10** with monosaccharide building block of formula **11** is performed in the presence of catalytic amounts of TMSOTf or TfOH in an aprotic apolar solvent at temperatures between -78 °C and -20 °C. Even more preferably, the coupling of monosaccharide building block of formula **10** with monosaccharide building block of formula **11** is performed in the presence of catalytic amounts of a Lewis acid in an aprotic apolar solvent at temperatures between -60 °C and -20 °C. Even more preferably, the coupling of monosaccharide building block of formula **10** with monosaccharide building block of formula **11** is performed in the presence of catalytic amounts of a Lewis acid in an aprotic apolar solvent at temperatures between -60 °C and 0 °C. Even more preferably, the coupling of monosaccharide building block of formula **10** with monosaccharide building block of formula **11** is performed in the presence of catalytic amounts of TMSOTf or TfOH in methylene chloride or toluene at -40 °C. According to the present invention the disaccharide of formula **2** is preferably obtained in form of crystals by recrystallization.

Thus, It is especially preferred that the method for the preparation of disaccharide of formula **2** comprises the following steps:
a2) Providing a monosaccharide building block of formula **10** wherein R¹ represents -Bz, R³ is a phenyl group and Z¹ is -O-C(=NPh)-CF₃; and a mono-saccharide building block **11** with R² is -Bn and X represents -SEt
b2) Coupling of the monosaccharide building block of formula **10** with the mono-saccharide building block of formula **11** in presence of catalytic amounts of TMSOTf or TfOH to yield disaccharide of formula **2.**

### Preparation of disaccharide of formula 3

Yet another aspect of the present invention relates to a method for preparation of a disaccharide of formula **3,** which comprises the following steps:
a6) Reacting the disaccharide of formula **2** with an alcohol HO-L-P in presence of a catalyst to yield a disaccharide of formula **9** 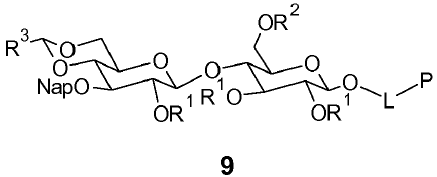 wherein
   R¹ represents an oxidation-stable and acid-stable protecting group,
   R² represents an oxidation-stable and acid-stable protecting group,
   R³ is an aryl or alkyl group,
   P is -NR'R" or -N₃,
   R and R represent independently of each other -Bn, -Cbz, -Allyl or R and R form together phthaloyl,
   L represents -(CH₂)ₙ-,
   n is an integer selected from 2, 3, 4, 5, 6, 7, 8, 9 and 10;
b6) Converting the disaccharide of formula **9** to the disaccharide of formula **3** by treatment with an oxidizing agent; 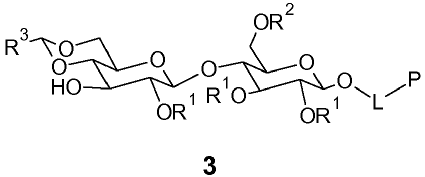 wherein
   R¹ represents an oxidation-stable and acid-stable protecting group,
   R² represents an oxidation-stable and acid-stable protecting group,
   R³ is an aryl or alkyl group,
   P is -NR'R" or -N₃,
   R and R represent independently of each other -Bn, -Cbz, -Allyl or R and R form together phthaloyl,
   L represents -(CH₂)ₙ-,
   n is an integer selected from 2, 3, 4, 5, 6, 7, 8, 9 and 10.

Preferably, the inventive method for preparation of a disaccharide of formula **3** comprises the following steps: a6) Reacting the disaccharide of formula **2** with an alcohol HO-L-P in presence of a catalyst to yield a disaccharide of formula **9** wherein R¹ is selected from -Bz, -ClAc, -Ac and -Piv, R² is selected from -Bz, - Ac, -Piv, -Bn and -TBS, R³ is selected from -Me, -Et, -Ph, -tBu and -PMP, P is -NR'R", R and R represent independently of each other -Bn, -Cbz, -Allyl or R and R form together phthaloyl, L represents -(CH₂)ₙ-, n is an integer selected from 2, 3, 4, 5, 6, 7, 8, 9 and 10;
b6) Converting the disaccharide of formula **9** to the disaccharide of formula **3** by treatment with an oxidizing agent.

More preferably, the inventive method for preparation of a disaccharide of formula 3 comprises the following steps a6) Reacting the disaccharide of formula **2** with an alcohol HO-L-P in presence of a catalyst to yield a disaccharide of formula **9** wherein R¹ represents -Bz, R² is selected from -Bz, -Ac, -Piv, -Bn and -TBS, R³ is selected from -Me, -Et, -Ph, -tBu and -PMP, P is -NR'R", R^{'} and R^{"} represent independently of each other -Bn, -Cbz, -Allyl or R and R form together phthaloyl, L represents -(CH₂)ₙ-, n is an integer selected from 2, 3, 4, 5, 6, 7, 8, 9 and 10; b6) Converting the disaccharide of formula **9** to the disaccharide of formula **3** by treatment with an oxidizing agent.

More preferably, the inventive method for preparation of a disaccharide of formula **3** comprises the following steps: a6) Reacting the disaccharide of formula **2** with an alcohol HO-L-P in presence of a catalyst to yield a disaccharide of formula **9** wherein R¹ is selected from -Bz, -ClAc, -Ac and -Piv, R² represents -Bn, R³ is selected from -Me, -Et, -Ph, -tBu and -PMP, P is -NR'R", R and R represent independently of each other -Bn, -Cbz, -Allyl or R and R form together phthaloyl, L represents -(CH₂)ₙ-, n is an integer selected from 2, 3, 4, 5, 6, 7, 8, 9 and 10; b6) Converting the disaccharide of formula **9** to the disaccharide of formula **3** by treatment with an oxidizing agent.

More preferably, the inventive method for preparation of a disaccharide of formula **3** comprises the following steps: a6) Reacting the disaccharide of formula **2** with an alcohol HO-L-P in presence of a catalyst to yield a disaccharide of formula **9** wherein R¹ is selected from -Bz, -ClAc, -Ac and -Piv, R² is selected from -Bz, - Ac, -Piv, -Bn and -TBS, R³ represents -Ph, P is -NR'R", R^{'} and R^{"} represent independently of each other -Bn, -Cbz, -Allyl or R and R form together phthaloyl, L represents -(CH₂)ₙ-, n is an integer selected from 2, 3, 4, 5, 6, 7, 8, 9 and 10; b6) Converting the disaccharide of formula **9** to the disaccharide of formula **3** by treatment with an oxidizing agent.

More preferably, the inventive method for preparation of a disaccharide of formula **3** comprises the following steps: a6) Reacting the disaccharide of formula **2** with an alcohol HO-L-P in presence of a catalyst to yield a disaccharide of formula **9** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, P is -N(Bn)Cbz, L represents -(CH₂)ₙ-, n is an integer selected from 2, 3, 4, 5, 6, 7, 8, 9 and 10; and b6) Converting the disaccharide of formula **9** to the disaccharide of formula **3** by treatment with an oxidizing agent.

More preferably, the inventive method for preparation of a disaccharide of formula **3** comprises the following steps: a6) Reacting the disaccharide of formula **2** with an alcohol HO-L-P in presence of a catalyst to yield a disaccharide of formula **9** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, P is -NR'R", R and R represent independently of each other -Bn, -Cbz, -Allyl or R and R form together phthaloyl, L represents -(CH₂)ₙ-, n is an integer selected from 2, 3, 4, and 5; b6) Converting the disaccharide of formula **9** to the disaccharide of formula **3** by treatment with an oxidizing agent.

More preferably, the inventive method for preparation of a disaccharide of formula **3** comprises the following steps: a6) Reacting the disaccharide of formula **2** with an alcohol HO-L-P in presence of a catalyst to yield a disaccharide of formula **9** wherein R¹ is selected from -Bz and -Ac, R² represents -Bn, R³ represents -Ph, P is -N(Bn)Cbz or -N₃, L represents -(CH₂)ₙ-, n is an integer selected from 2, 3, 4 and 5; b6) Converting the disaccharide of formula **9** to the disaccharide of formula **3** by treatment with an oxidizing agent.

It is preferred that the reaction of disaccharide of formula **2** with an alcohol HO-L-P to disaccharide of formula **9** is performed in the presence of NIS and catalytic amounts of a strong acid in an aprotic apolar solvent at low temperatures between -30 °C and 10 °C. It is more preferred that the reaction of disaccharide of formula **2** with an alcohol HO-L-P to disaccharide of formula **9** is performed in the presence of NIS and catalytic amounts of trifluoromethanesulfonic acid in an aprotic apolar solvent at low temperatures between -30 °C and 10 °C. It is more preferred that the reaction of disaccharide of formula **2** with an alcohol HO-L-P to disaccharide of formula **9** is performed in the presence of NIS and catalytic amounts of trifluoromethanesulfonic acid in methylene chloride at low temperatures between -30 °C and 10 °C. It is even more preferred that the reaction of disaccharide of formula **2** with an alcohol HO-L-P to disaccharide of formula **9** is performed in the presence of NIS and catalytic amounts of trifluoromethanesulfonic acid in methylene chloride at low temperatures between -20 °C and 0 °C. It is especially preferred that the reaction of disaccharide of formula **2** with an alcohol HO-L-P to disaccharide of formula **9** is performed in the presence of NIS and catalytic amounts of trifluoromethanesulfonic acid in methylene chloride at low temperatures between -10 °C and 0 °C. According to the present invention the disaccharide of formula **9** is preferably obtained in form of crystals by recrystallization
Preferably, the conversion of disaccharide of formula **9** to disaccharide of formula **3** is performed by treatment with an oxidizing agent in an aprotic apolar solvent at lowered temperatures between -10 °C and 20 °C. More preferably, the conversion of disaccharide of formula **9** to disaccharide of formula **3** is performed by treatment with DDQ in an aprotic apolar solvent at lowered temperatures between -10 °C and 20 °C. More preferably, the conversion of disaccharide of formula **9** to disaccharide of formula **3** is performed by treatment with DDQ in methylene chloride at lowered temperatures between -10 °C and 20 °C. Even more preferably, the conversion of disaccharide of formula **9** to disaccharide of formula **3** is performed by treatment with DDQ in methylene chloride at lowered temperatures between 0 °C and 10 °C.
Thus, it is especially preferred that the inventive method for preparation of a disaccharide of formula **3** comprises the following steps: a6) Reacting the disaccharide of formula **2** with an alcohol HO-L-P ; in presence of NIS and catalytic amounts of trifluoromethanesulfonic acid to yield a disaccharide of formula **9** wherein R¹ represents -Bz, R² is -Bn, R³ is a phenyl group, P is -N(Bn)Cbz, L represents -(CH₂)ₙ-, n is an integer selected from 2, 3, 4 and 5; and b6) Converting the disaccharide of formula **9** to the disaccharide of formula **3** by treatment with DDQ.

### Coupling of disaccharide of formula 2 and disaccharide of formula 3 to protected tetrasaccharide of formula 1

Another aspect of the present invention relates to a synthetic method for preparation of protected tetrasaccharide of formula **1,** comprising the following steps:
A) Providing a disaccharide of formula **2** 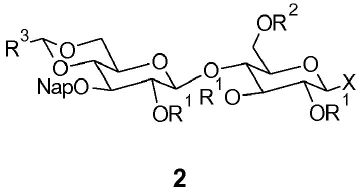 wherein
   R¹ represents an oxidation-stable and acid-stable protecting group,
   R² represents an oxidation-stable and acid-stable protecting group,
   R³ is an aryl or alkyl group, and
   X is a leaving group;
      and a disaccharide of formula **3** 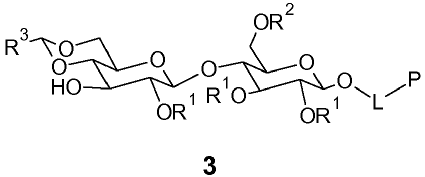
   wherein
   R¹ represents an oxidation-stable and acid-stable protecting group,
   R² represents an oxidation-stable and acid-stable protecting group,
   R³ is an aryl or alkyl group,
   P is -NR'R" or -N₃,
   R and R represent independently of each other -Bn, -Cbz, -Allyl or
   R and R form together phthaloyl,
   L represents -(CH₂)ₙ-,
   n is an integer selected from 2, 3, 4, 5, 6, 7, 8, 9 and 10;
B) Coupling of the disaccharide of formula **2** with the disaccharide of formula 3 in the presence of a catalyst to obtain a tetrasaccharide of formula **1** 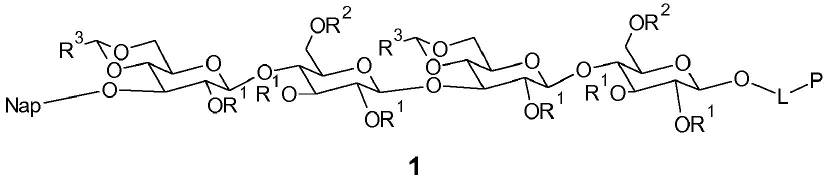 wherein
   R¹ represents an oxidation-stable and acid-stable protecting group,
   R² represents an oxidation-stable and acid-stable protecting group,
   R³ is an aryl or alkyl group,
   P is -NR'R" or -N₃,
   R and R represent independently of each other -Bn, -Cbz, -Allyl or
   R and R form together phthaloyl,
   L represents -(CH₂)ₙ-,
   n is an integer selected from 2, 3, 4, 5, 6, 7, 8, 9 and 10.

It is preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, X represents -SR⁵ and R⁵ is selected from -Me, -Et, -Pr, -Bu, -Ph, and -Tol and a disaccharide of formula **3** with P is -NR'R", R and R represent independently of each other -Bn, -Cbz, -Allyl or R and R form together phthaloyl, L represents -(CH₂)ₙ-, n is an integer selected from 2, 3, 4, 5, 6, 7, 8, 9 and 10; B) Coupling of the disaccharide of formula **2** with the disaccharide of formula **3** in the presence of a catalyst to obtain a tetrasaccharide of formula **1.**

It is more preferred that the inventive method comprises the following steps:
A) Providing a disaccharide of formula **2** wherein R¹ represents -Bz, R² is selected from -Bz, -Ac, -Piv, -Bn and -TBS, R³ is selected from -Me, -Et, -Ph, -tBu and -PMP, X represents -SR⁵, and R⁵ is selected from -Me, -Et, -Pr, -Bu, -Ph, and -Tol and a disaccharide of formula **3** with P is -NR'R", R and R represent independently of each other -Bn, -Cbz, -Allyl or R and R form together phthaloyl, L represents -(CH₂)ₙ-, n is an integer selected from 2, 3, 4, 5, 6, 7, 8, 9 and 10; B) Coupling of the disaccharide of formula **2** with the disaccharide of formula **3** in the presence of a catalyst to obtain a tetrasaccharide of formula **1.**

It is more preferred that the inventive method comprises the following steps:
A) Providing a disaccharide of formula **2** wherein R¹ is selected from -Bz, -ClAc, -Ac and -Piv, R² represents -Bn, R³ is selected from -Me, -Et, -Ph, -tBu or -PMP, X represents -SR⁵ and R⁵ is selected from -Me, -Et, -Pr, -Bu, -Ph, and -Tol and a disaccharide of formula **3** with P is -NR'R", R and R represent independently of each other -Bn, -Cbz, -Allyl or R and R form together phthaloyl, L represents -(CH₂)ₙ-, n is an integer selected from 2, 3, 4, 5, 6, 7, 8, 9 and 10;
B) Coupling of the disaccharide of formula **2** with the disaccharide of formula **3** in the presence of a catalyst to obtain a tetrasaccharide of formula **1.**

It is more preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ represents -Ph, X represents -SR⁵ and R⁵ is selected from -Me, -Et, -Pr, -Bu, -Ph, and -Tol and a disaccharide of formula **3** with P is -NR'R", R and R represent independently of each other -Bn, -Cbz, -Allyl or R and R form together phthaloyl, L represents -(CH₂)ₙ-, n is an integer selected from 2, 3, 4, 5, 6, 7, 8, 9 and 10;
B) Coupling of the disaccharide of formula **2** with the disaccharide of formula **3** in the presence of a catalyst to obtain a tetrasaccharide of formula **1.**

It is even more preferred that the inventive method comprises the following steps:
A) Providing a disaccharide of formula **2** wherein R¹ is selected from -Bz, -ClAc, -Ac or -Piv, R² is selected from -Bz, -Ac, -Piv, -Bn and -TBS, R³ is selected from -Me, -Et, -Ph, -tBu and -PMP, X represents -SEt and a disaccharide of formula **3** with P is -NR'R", R and R represent independently of each other -Bn, -Cbz, -Allyl or R and R form together phthaloyl, L represents -(CH₂)ₙ-, n is an integer selected from 2, 3, 4, 5, 6, 7, 8, 9 and 10; B) Coupling of the disaccharide of formula **2** with the disaccharide of formula **3** in the presence of a catalyst to obtain a tetrasaccharide of formula **1.**

It is even more preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, X represents -SR⁵ and R⁵ is selected from -Me, -Et, -Pr, -Bu, -Ph, and -Tol and a disaccharide of formula **3** with P is -N(Bn)Cbz, L represents -(CH₂)ₙ-, n is an integer selected from 2, 3, 4, 5, 6, 7, 8, 9 and 10; B) Coupling of the disaccharide of formula **2** with the disaccharide of formula **3** in the presence of a catalyst to obtain a tetrasaccharide of formula **1.**

It is even more preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, X represents -SR⁵ and R⁵ is selected from -Me, -Et, -Pr, -Bu, -Ph, and -Tol and a disaccharide of formula **3** with P is -NR'R", R and R represent independently of each other -Bn, -Cbz, -Allyl or R and R form together phthaloyl, L represents -(CH₂)ₙ-, n is an integer selected from 2, 3, 4, and 5; B) Coupling of the disaccharide of formula **2** with the disaccharide of formula **3** in the presence of a catalyst to obtain a tetrasaccharide of formula **1.**

It is even more preferred that the inventive method comprises the following steps:
A) Providing a disaccharide of formula **2** wherein R¹ is selected from -Bz and -Ac, R² represents -Bn, R³ represents -Ph, X represents -SEt and a disaccharide of formula **3** wherein P is -N(Bn)Cbz or N₃, L represents -(CH₂)ₙ-, n is an integer selected from 2, 3, 4, and 5; B) Coupling of the disaccharide of formula **2** with the disaccharide of formula **3** in the presence of a catalyst to obtain a tetrasaccharide of formula **1.**

Preferably, the coupling reaction of disaccharide of formula **2** with the disaccharide of formula **3** is performed in the presence of NIS and catalytic amounts of a strong acid in an aprotic apolar solvent at low temperatures between -20 °C and 10 °C. More preferably, the coupling reaction of disaccharide of formula **2** with the disaccharide of formula **3** is performed in the presence of NIS and catalytic amounts of trifluoromethanesulfonic acid in an aprotic apolar solvent at low temperatures between -20 °C and 10 °C. More preferably, the coupling reaction of disaccharide of formula **2** with the disaccharide of formula **3** is performed in the presence of NIS and catalytic amounts of trifluoromethanesulfonic acid in methylene chloride at low temperatures between -20 °C and 5 °C. Even more preferably, the coupling reaction of disaccharide of formula **2** with the disaccharide of formula **3** is performed in the presence of NIS and catalytic amounts of trifluoromethanesulfonic acid in methylene chloride at low temperatures between -10 °C and 0 °C. According to the present invention the protected tetrasaccharide of formula **1** is preferably obtained in form of crystals by recrystallization or trituration.

Thus, it is especially preferred that the inventive method for preparation of protected tetrasaccharide of formula **1** comprises the following steps:
A) Providing a disaccharide of formula **2** with R¹ is -Bz, R² is -Bn, R³ is a phenyl group, X represents -SEt and a disaccharide of formula **3** with P is -N(Bn)Cbz, L represents -(CH₂)ₙ-, n is an integer selected from 2, 3, 4 and 5;
B) Coupling of the disaccharide of formula **2** with the disaccharide of formula **3** in the presence of NIS and catalytic amounts of trifluoromethanesulfonic acid to obtain a tetrasaccharide of formula **1.**

### Preparation of tetrasaccharide of formula 4

Still a further aspect of the present invention relates to a synthetic method for preparation of tetrasaccharide of formula **4,** which comprises the following step: Performing selective removal of acetal groups by treatment of the tetrasaccharide of formula **1** with a nucleophile in presence of a catalytic amount of an acid to yield a tetrasaccharide of formula **4,** 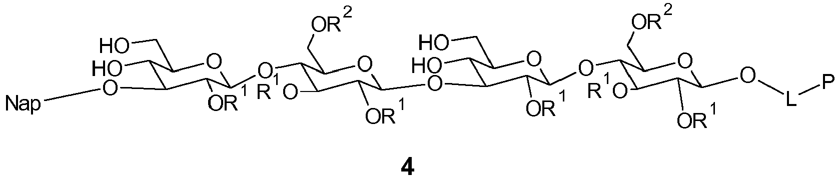 wherein
R¹ represents an oxidation-stable and acid-stable protecting group,
R² represents an oxidation-stable and acid-stable protecting group,
P is -NR'R" or -N₃,
R and R represent independently of each other -Bn, -Cbz, -Allyl or
R and R form together phthaloyl,
L represents -(CH₂)ₙ-,
n is an integer selected from 2, 3, 4, 5, 6, 7, 8, 9 and 10.

Preferably the inventive method for preparation of tetrasaccharide of formula **4** comprises the following step: Performing selective removal of acetal groups by treatment of the tetrasaccharide of formula **1** with a nucleophile in presence of a catalytic amount of an acid to yield a tetrasaccharide of formula **4,** wherein R¹ is selected from -Bz, -ClAc, -Ac and -Piv, R² is selected from -Bz, -Ac, -Piv, -Bn and -TBS, P is -NR'R", R and R represent independently of each other -Bn, -Cbz, -Allyl or R and R form together phthaloyl, L represents -(CH₂)ₙ- and n is an integer selected from 2, 3, 4, 5, 6, 7, 8, 9 and 10.

More preferably the inventive method for preparation of tetrasaccharide of formula **4** comprises the following step: Performing selective removal of acetal groups by treatment of the tetrasaccharide of formula **1** with a nucleophile in presence of a catalytic amount of an acid to yield a tetrasaccharide of formula **4,** wherein R¹ represents -Bz, R² is selected from -Bz, -Ac, -Piv, -Bn and -TBS, P is -NR'R", R and R represent independently of each other -Bn, -Cbz, -Allyl or R and R form together phthaloyl, L represents -(CH₂)ₙ- and n is an integer selected from 2, 3, 4, 5, 6, 7, 8, 9 and 10.

More preferably the inventive method for preparation of tetrasaccharide of formula **4** comprises the following step: Performing selective removal of acetal groups by treatment of the tetrasaccharide of formula **1** with a nucleophile in presence of a catalytic amount of an acid to yield a tetrasaccharide of formula **4,** wherein R¹ is selected from -Bz, -ClAc, -Ac and -Piv, R² represents -Bn, P is -NR'R", R^{'} and R represent independently of each other -Bn, -Cbz, -Allyl or R and R form together phthaloyl, L represents -(CH₂)ₙ- and n is an integer selected from 2, 3, 4, 5, 6, 7, 8, 9 and 10.

More preferably the inventive method for preparation of tetrasaccharide of formula **4** comprises the following step: Performing selective removal of acetal groups by treatment of the tetrasaccharide of formula **1** with a nucleophile in presence of a catalytic amount of an acid to yield a tetrasaccharide of formula **4,** wherein R¹ is selected from -Bz, -ClAc, -Ac and -Piv, R² is selected from -Bz, -Ac, -Piv, -Bn and -TBS, P is -N(Bn)Cbz, L represents -(CH₂)ₙ- and n is an integer selected from 2, 3, 4, 5, 6, 7, 8, 9 and 10.

Even more preferably the inventive method for preparation of tetrasaccharide of formula **4** comprises the following step: Performing selective removal of acetal groups by treatment of the tetrasaccharide of formula **1** with a nucleophile in presence of a catalytic amount of an acid to yield a tetrasaccharide of formula **4,** wherein R¹ is selected from -Bz, -ClAc, -Ac and -Piv, R² is selected from -Bz, -Ac, -Piv, -Bn and -TBS, P is -NR'R", R and R represent independently of each other -Bn, -Cbz, -Allyl or R and R form together phthaloyl, L represents -(CH₂)ₙ- and n is an integer selected from 2, 3, 4 and 5.

Even more preferably the inventive method for preparation of tetrasaccharide of formula **4** comprises the following step: Performing selective removal of acetal groups by treatment of the tetrasaccharide of formula **1** with a nucleophile in presence of a catalytic amount of an acid to yield a tetrasaccharide of formula **4,** wherein R¹ is selected from -Bz and -Ac, R² represents -Bn, P is -N(Bn)Cbz or N₃, L represents -(CH₂)ₙ- and n is an integer selected from 2, 3, 4 and 5.

It is preferred that the selective removal of acetal groups of tetrasaccharide of formula **1** is performed in the presence of catalytic amounts of an acid and a nucleophile, such as a thiol, water or an alcohol. It is even more preferred that the selective removal of acetal groups of tetrasaccharide of formula **1** is performed in the presence of catalytic amounts of a sulfonic acid and a thiol. It is even more preferred that the selective removal of acetal groups of tetrasaccharide of formula **1** is performed in the presence of catalytic amounts of PTSA and a thiol. It is especially preferred that the selective removal of acetal groups of tetrasaccharide of formula **1** is performed in the presence of catalytic amounts of PTSA and ethanethiol. According to the present invention the tetrasaccharide of formula **4** is preferably obtained in form of crystals by recrystallization or trituration.

Thus, it is especially preferred that the inventive method for preparation of tetrasaccharide of formula **4** comprises the following step: Performing selective removal of acetal groups by treatment of the tetrasaccharide of formula **1** with ethanethiol in presence of a catalytic amount of PTSA to yield a tetrasaccharide of formula **4,** wherein R¹ is -Bz, R² is -Bn, P is -N(Bn)CBz, L represents -(CH₂)ₙ- and n is an integer selected from 2, 3, 4 and 5.

### Preparation of tetrasaccharide diacid of formula 5

A further aspect of the present invention relates to a synthetic method for preparation of tetrasaccharide diacid of formula **5,** which comprises the following step:

Performing oxidation of the tetrasaccharide of formula **4** to yield a tetrasaccharide diacid of formula **5,** 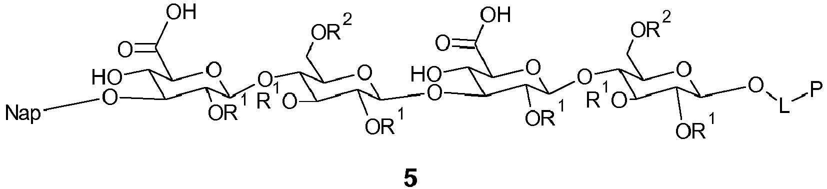 wherein
R¹ represents an oxidation-stable and acid-stable protecting group,
R² represents an oxidation-stable and acid-stable protecting group,
P is -NR'R" or -N₃,
R and R represent independently of each other -Bn, -Cbz, -Allyl or
R and R form together phthaloyl,
L represents -(CH₂)ₙ-,
n is an integer selected from 2, 3, 4, 5, 6, 7, 8, 9 and 10.

Preferably the inventive method for preparation of tetrasaccharide diacid of formula **5** comprises the following step: Performing oxidation of the tetrasaccharide of formula **4** to yield a tetrasaccharide diacid of formula **5,** wherein R¹ is selected from -Bz, -ClAc, -Ac, and -Piv, R² is selected from -Bz, -Ac, -Piv, -Bn and -TBS, P is -NR'R", R and R represent independently of each other -Bn, -Cbz, -Allyl or R and R form together phthaloyl, L represents -(CH₂)ₙ- and n is an integer selected from 2, 3, 4, 5, 6, 7, 8, 9 and 10.

More preferably the inventive method for preparation of tetrasaccharide diacid of formula **5** comprises the following step: Performing oxidation of the tetrasaccharide of formula **4** to yield a tetrasaccharide diacid of formula **5,** wherein R¹ represents -Bz, R² is selected from -Bz, -Ac, -Piv, -Bn and -TBS, P is -NR'R", R and R represent independently of each other -Bn, -Cbz, -Allyl or R and R form together phthaloyl, L represents -(CH₂)ₙ- and n is an integer selected from 2, 3, 4, 5, 6, 7, 8, 9 and 10.

More preferably the inventive method for preparation of tetrasaccharide diacid of formula **5** comprises the following step: Performing oxidation of the tetrasaccharide of formula **4** to yield a tetrasaccharide diacid of formula **5,** wherein R¹ is selected from -Bz, -ClAc, -Ac, and -Piv, R² represents -Bn, P is -NR'R", R^{'} and R represent independently of each other -Bn, -Cbz, -Allyl or R and R form together phthaloyl, L represents -(CH₂)ₙ- and n is an integer selected from 2, 3, 4, 5, 6, 7, 8, 9 and 10.

More preferably the inventive method for preparation of tetrasaccharide diacid of formula **5** comprises the following step: Performing oxidation of the tetrasaccharide of formula **4** to yield a tetrasaccharide diacid of formula **5,** wherein R¹ is selected from -Bz, -ClAc, -Ac, and -Piv, R² is selected from -Bz, -Ac, -Piv, -Bn and -TBS, P is -N(Bn)Cbz, L represents -(CH₂)ₙ- and n is an integer selected from 2, 3, 4, 5, 6, 7, 8, 9 and 10.

Even more preferably the inventive method for preparation of tetrasaccharide diacid of formula **5** comprises the following step: Performing oxidation of the tetrasaccharide of formula **4** to yield a tetrasaccharide diacid of formula **5,** wherein R¹ is selected from -Bz, -ClAc, -Ac, and -Piv, R² is selected from -Bz, -Ac, -Piv, -Bn and -TBS, P is -NR'R", R and R represent independently of each other -Bn, -Cbz, -Allyl or R and R form together phthaloyl, L represents -(CH₂)ₙ- and n is an integer selected from 2, 3, 4 and 5.

Even more preferably the inventive method for preparation of tetrasaccharide diacid of formula **5** comprises the following step: Performing oxidation of the tetrasaccharide of formula **4** to yield a tetrasaccharide diacid of formula **5,** wherein R¹ is selected from -Bz and -Ac, R² represents -Bn, P is -N(Bn)Cbz or N₃, L represents -(CH₂)ₙ- and n is an integer selected from 2, 3, 4 and 5.

It is preferred that the oxidation of tetrasaccharide of formula **4** is performed in the presence of at least one oxidizing agent in a mixture of water and an aprotic apolar solvent at low temperatures between -10 °C and 10 °C. It is even more preferred that the oxidation of tetrasaccharide of formula **4** is performed in the presence of a catalytic amount of a first oxidizing agent and a second oxidizing agent in a mixture of water and an aprotic apolar solvent at low temperatures between -10 °C and 10 °C. It is even more preferred that the oxidation of tetrasaccharide of formula **4** is performed in the presence of a catalytic amount of TEMPO and a second oxidizing agent in a mixture of water and an aprotic apolar solvent at low temperatures between -10 °C and 10 °C. It is even more preferred that the oxidation of tetrasaccharide of formula **4** is performed in the presence of a catalytic amount of TEMPO and bis(acetoxy)iodobenzene (BAIB) in a mixture of water and an aprotic apolar solvent at low temperatures between -10 °C and 10 °C. It is even more preferred that the oxidation of tetrasaccharide of formula **4** is performed in the presence of a catalytic amount of TEMPO and bis(acetoxy)iodobenzene (BAIB) in a mixture of water and methylene chloride at low temperatures between - 10 °C and 10 °C. It is especially preferred that the oxidation of tetrasaccharide of formula **4** is performed in the presence of a catalytic amount of TEMPO and bis(acetoxy)iodobenzene (BAIB) in a mixture of water and methylene chloride at 0 °C. According to the present invention the tetrasaccharide diacid of formula **5** is preferably obtained in form of crystals by recrystallization or trituration.

Thus, it is especially preferred that the inventive method for preparation of tetrasaccharide diacid of formula **5** comprises the following step: Performing oxidation of the tetrasaccharide of formula **4** with a catalytic amount of TEMPO and BAIB to yield a tetrasaccharide diacid of formula **5,** wherein R¹ is -Bz, R² is -Bn, P is -N(Bn)CBz, L represents -(CH₂)ₙ- and n is an integer selected from 2, 3, 4 and 5.

### Preparation of tetrasaccharide of formula 7

In another aspect of the present invention, the inventive method for preparation of tetrasaccharide of formula **7** comprises the following step: Performing removal of protecting groups R¹ of tetrasaccharide of formula **5** in presence of a base to yield tetrasaccharide of formula **7,** 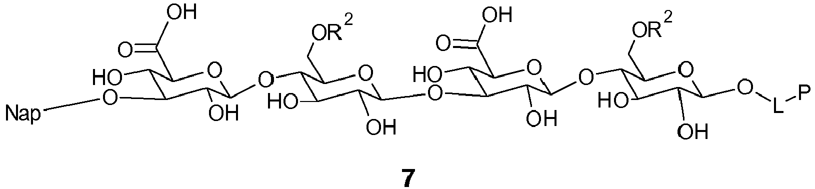 wherein
R¹ represents an oxidation-stable and acid-stable protecting group,
R² represents an oxidation-stable and acid-stable protecting group,
P is -NR'R" or -N₃,
R and R represent independently of each other -Bn, -Cbz, -Allyl or
R and R form together phthaloyl,
L represents -(CH₂)ₙ-,
n is an integer selected from 2, 3, 4, 5, 6, 7, 8, 9 and 10.

It is preferred that the inventive method for preparation of tetrasaccharide of formula **7** comprises the following step: Performing removal of protecting groups R¹ of tetrasaccharide of formula **5** in presence of a base to yield tetrasaccharide of formula **7,** wherein R¹ is selected from -Bz, -ClAc, -Acand -Piv, R² is selected from -Bz, -Ac, -Piv, -Bn and -TBS, P is -NR'R", R and R represent independently of each other -Bn, -Cbz, -Allyl or R and R form together phthaloyl, L represents -(CH₂)ₙ- and n is an integer selected from 2, 3, 4, 5, 6, 7, 8, 9 and 10.

It is more preferred that the inventive method for preparation of tetrasaccharide of formula **7** comprises the following step: Performing removal of protecting groups R¹ of tetrasaccharide of formula **5** in presence of a base to yield tetrasaccharide of formula **7,** wherein R¹ represents -Bz, R² is selected from -Bz, -Ac, -Piv, -Bn and -TBS, P is -NR'R", R and R represent independently of each other -Bn, -Cbz, -Allyl or R and R form together phthaloyl, L represents -(CH₂)ₙ- and n is an integer selected from 2, 3, 4, 5, 6, 7, 8, 9 and 10.

It is more preferred that the inventive method for preparation of tetrasaccharide of formula **7** comprises the following step: Performing removal of protecting groups R¹ of tetrasaccharide of formula **5** in presence of a base to yield tetrasaccharide of formula **7,** wherein R¹ is selected from -Bz, -ClAc, -Ac and -Piv, R² represents -Bn, P is -NR'R", R and R represent independently of each other -Bn, -Cbz, -Allyl or R and R form together phthaloyl, L represents -(CH₂)ₙ- and n is an integer selected from 2, 3, 4, 5, 6, 7, 8, 9 and 10.

It is even more preferred that the inventive method for preparation of tetrasaccharide of formula **7** comprises the following step: Performing removal of protecting groups R¹ of tetrasaccharide of formula **5** in presence of a base to yield tetrasaccharide of formula **7,** wherein R¹ is selected from -Bz, -ClAc, -Ac and -Piv, R² is selected from -Bz, -Ac, -Piv, -Bn and -TBS, P is -N(Bn)Cbz, L represents -(CH₂)ₙ- and n is an integer selected from 2, 3, 4, 5, 6, 7, 8, 9 and 10.

It is even more preferred that the inventive method for preparation of tetrasaccharide of formula **7** comprises the following step: Performing removal of protecting groups R¹ of tetrasaccharide of formula **5** in presence of a base to yield tetrasaccharide of formula **7,** wherein R¹ is selected from -Bz, -ClAc, -Ac and -Piv, R² is selected from -Bz, -Ac, -Piv, -Bn and -TBS, P is -NR'R", R^{'} and R^{"} represent independently of each other -Bn, -Cbz, -Allyl or R and R form together phthaloyl, L represents -(CH₂)ₙ- and n is an integer selected from 2, 3, 4 and 5.

It is even more preferred that the inventive method for preparation of tetrasaccharide of formula **7** comprises the following step: Performing removal of protecting groups R¹ of tetrasaccharide of formula **5** in presence of a base to yield tetrasaccharide of formula **7,** wherein R¹ is selected from -Bz and -Ac, R² represents -Bn, P is -N(Bn)Cbz or N₃, L represents -(CH₂)ₙ- and n is an integer selected from 2, 3, 4 and 5.

Preferably, the removal of protecting groups R¹ of tetrasaccharide of formula 5 to yield tetrasaccharide of formula **7** is performed in presence of a base in a protic solvent at elevated temperatures between 25 °C and 60 °C. More preferably, the removal of protecting groups R¹ of tetrasaccharide of formula **5** to yield tetrasaccharide of formula **7** is performed in presence of sodium methoxide in a protic solvent at elevated temperatures between 25 °C and 60 °C. More preferably, the removal of protecting groups R¹ of tetrasaccharide of formula **5** to yield tetrasaccharide of formula **7** is performed in presence of sodium methoxide in an alcohol at elevated temperatures between 30 °C and 50 °C. Even more preferably, the removal of protecting groups R¹ of tetrasaccharide of formula 5 to yield tetrasaccharide of formula **7** is performed in presence of sodium methoxide in methanol at 40 °C. According to the present invention the tetrasaccharide of formula **7** is preferably obtained in form of crystals by recrystallization or trituration.

Thus, it is especially preferred that the inventive method for preparation of tetrasaccharide of formula **7** comprises the following step: Performing removal of protecting groups R¹ of tetrasaccharide of formula **5** in presence of sodium methoxide in methanol to yield tetrasaccharide of formula **7,** wherein R¹ is -Bz, R² is -Bn, P is -N(Bn)CBz, L represents -(CH₂)ₙ- and n is an integer selected from 2, 3, 4 and 5.

### Preparation of tetrasaccharide of formula 8

Yet another aspect of the present invention is the synthetic method for preparation of tetrasaccharide of formula **8,** which comprises the following step: Hydrogenating the tetrasaccharide of formula **7** in presence of a catalyst to yield a tetrasaccharide of formula **8,** 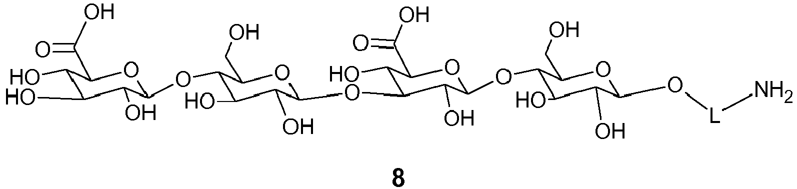
wherein L represents -(CH₂)ₙ- and
n is an integer selected from 2, 3, 4, 5, 6, 7, 8, 9 and 10.

Preferably, the inventive method for preparation of tetrasaccharide of formula **8** comprises the following step: Hydrogenating the tetrasaccharide of formula **7** in presence of a catalyst to yield a tetrasaccharide of formula **8,** wherein L represents -(CH₂)ₙ- and n is an integer selected from 2, 3, 4, 5, 6, 7 and 8. More preferably, the inventive method for preparation of tetrasaccharide of formula 8 comprises the following step: Performing a hydrogenation reaction of the tetrasaccharide of formula **7** in the presence of a catalyst to yield tetrasaccharide of formula **8,** wherein L represents -(CH₂)ₙ- and n is an integer selected from 2, 3, 4, and 5.

It is preferred that the hydrogenation reaction of the tetrasaccharide of formula 7 to yield tetrasaccharide of formula **8** is performed in the presence of a catalyst in a mixture of an alcohol and an aprotic apolar solvent. It is more preferred that the hydrogenation reaction of the tetrasaccharide of formula **7** to yield tetrasaccharide of formula **8** is performed in the presence of a palladium catalyst in a mixture of an alcohol and an aprotic apolar solvent It is even more preferred that the hydrogenation reaction of the tetrasaccharide of formula **7** to yield tetrasaccharide of formula **8** is performed in the presence of palladium on carbon (Pd/C) and/or Pd(OH)₂/C in a mixture of an alcohol and an aprotic apolar solvent. It is even more preferred that the hydrogenation reaction of the tetrasaccharide of formula **7** to yield tetrasaccharide of formula **8** is performed in the presence of palladium on carbon (Pd/C) and/or Pd(OH)₂/C in a mixture of tBuOH and methylene chloride.

Thus, it is especially preferred that the inventive method for preparation of tetrasaccharide of formula **8** comprises the following step: Hydrogenating the tetrasaccharide of formula **7** in the presence of Pd/C and/or Pd(OH)₂/C to yield a tetrasaccharide of formula **8,** wherein L represents -(CH₂)ₙ- and n is an integer selected from 2, 3, 4, and 5.

The present invention provides very favorable and efficient methods for synthesizing the tetrasaccharide of formula **8** and tetrasaccharide conjugates selectively and in high yields.

These methods involve the use of one or more of molecules of general formula **1', 2a, 4', 5', 7', 9'** and **10a,** wherein n is an integer selected from 2, 3, 4, 5, 6, 7, 8, 9 and 10, as shown or defined below. Preferably n is an integer selected from 2, 3, 4 and 5. Especially preferred is, when n is selected from 2 and 5.

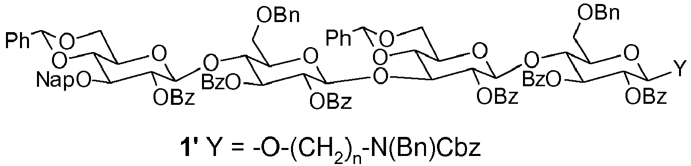

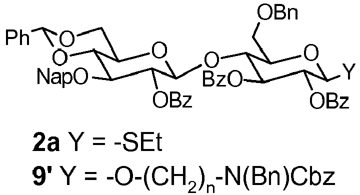

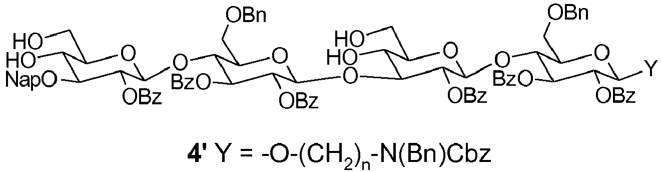

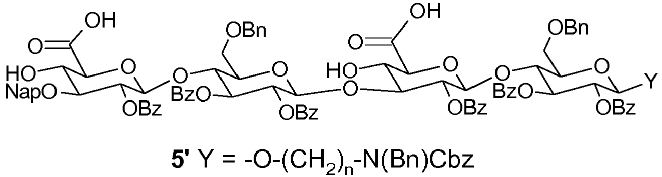

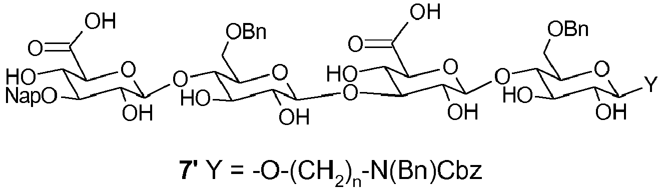

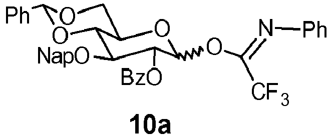

### ST3 tetrasaccharide conjugates

The method for preparing the ST3 tetrasaccharide conjugate of the present invention typically comprises coupling the ST3 tetrasaccharide of the invention bearing a linker or spacer group, in particular wherein the linker is -(CH₂)ₙ-NH₂, with n being an integer from 2 to 10, preferably from 2 to 5, i.e. tetrasaccharide of formula **8,** with a carrier protein or a glycosphingolipid.

More specifically, the method for preparing the ST3 tetrasaccharide conjugate of the present invention typically comprises the following two steps:
a4) reacting the tetrasaccharide of formula **8** wherein L is -(CH₂)ₙ-, with n being selected from 2, 3, 4, 5, 6, 7, 8, 9 and 10; with a linker J¹-L²-J² under addition of a base to yield tetrasaccharide of formula **16,** 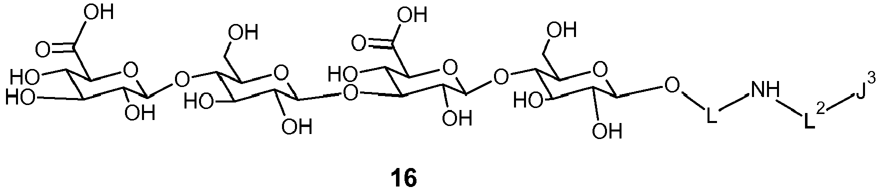
   wherein L represents -(CH₂)ₙ-,
   n is an integer selected from 2, 3, 4, 5, 6, 7, 8, 9 and 10,
   J³ is J¹ or J²,
   L² is selected from: 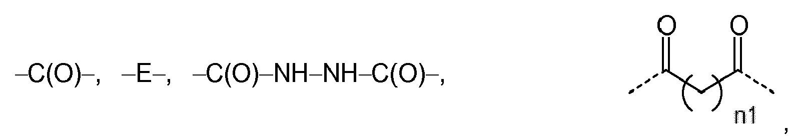 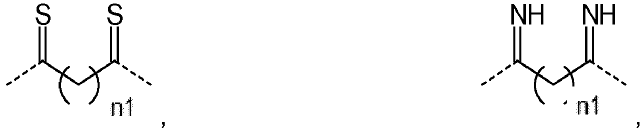 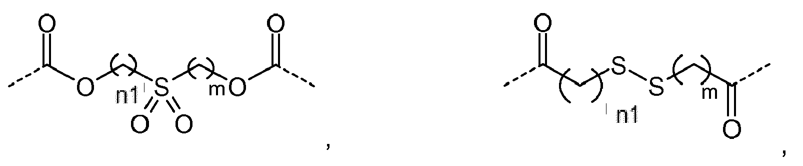 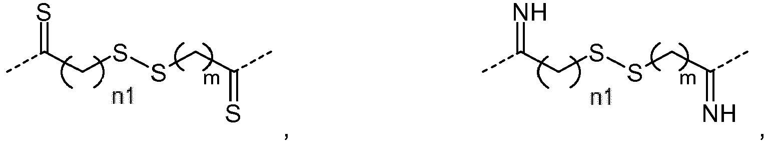 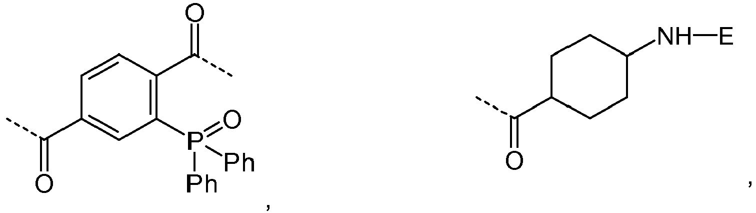 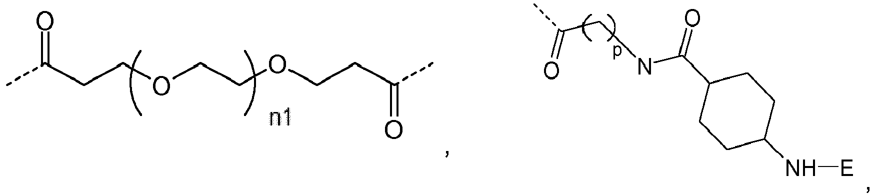 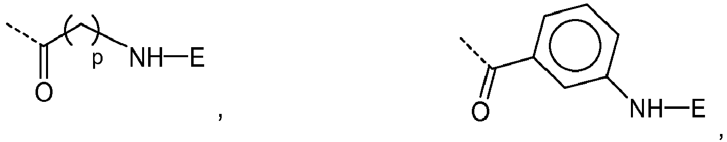 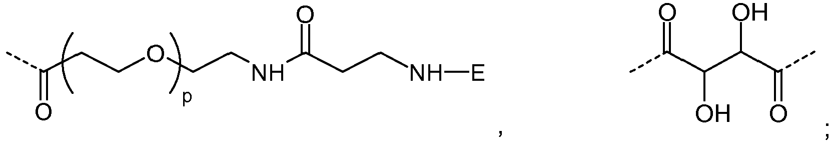
   E is selected from 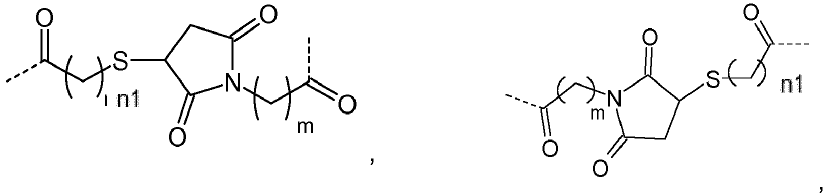 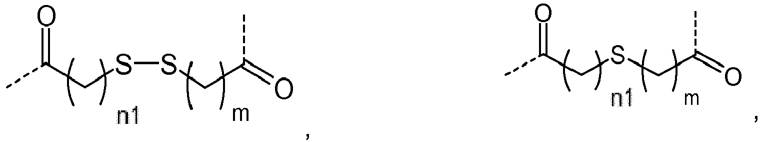 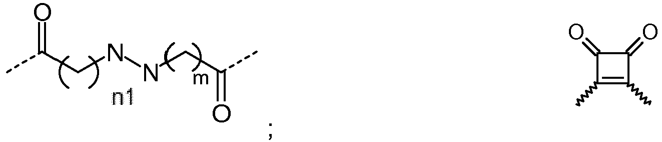
   m, n1, o and p represent independently of each other an integer from 1 to 10 and
   J¹ and J² are selected independently of each other from 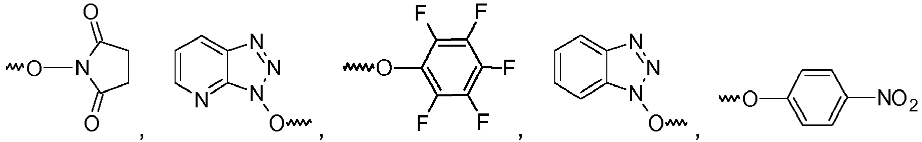
and b4) coupling the tetrasaccharide of formula **16** to a carrier protein or a glycosphingolipid.

More preferably, the method for preparing the ST3 tetrasaccharide conjugate of the present invention comprises a4) reacting the tetrasaccharide of formula **8** wherein L is -(CH₂)ₙ-, with n being selected from 2, 3, 4, and 5; with the linker di(N-succinimidyl) adipate under addition of a base to yield tetrasaccharide of formula **16,** and b4) coupling the tetrasaccharide of formula **16** to a carrier protein or a glycosphingolipid

In one preferred embodiment according to the present invention said method for preparing the ST3 tetrasaccharide conjugate comprises a4) reacting the tetrasaccharide of formula **8** wherein L is -(CH₂)ₙ-, with n being selected from 2, 3, 4, and 5; with the linker di(N-succinimidyl) adipate under addition of a base to yield tetrasaccharide of formula **16,** and b4) coupling the tetrasaccharide of formula **16** to a carrier protein.

In an even more preferred embodiment according to the present invention said method for preparing the ST3 tetrasaccharide conjugate comprises a4) reacting the tetrasaccharide of formula **8** wherein L is -(CH₂)ₙ-, with n being selected from 2, 3, 4, and 5; with the linker di(N-succinimidyl) adipate under addition of triethylamine to yield tetrasaccharide of formula **16,** and b4) coupling the tetrasaccharide of formula **16** to a CRM₁₉₇ carrier protein.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### Abbreviations

- NIS: N-iodosuccinimide;
- TfOH: triflic acid
- h: hour(s)
- d: day(s)
- DCM: dichloromethane
- TLC: thin layer chromatography
- MS: molecular sieves
- TEMPO: 2,2,6,6-tetramethyl-1-piperidinyloxy, free radical
- Cbz: carbonyloxybenzyl
- Nap: 2-naphthylmethyl
- BAIB: bis(acetoxy)iodobenzene
- PTSA: para-toluenesulfonic acid
- PMP: para-methoxyphenyl

### General information

Commercial grade solvents were used unless stated otherwise. Dry solvents were obtained from a Waters Dry Solvent System. Solvents for chromatography were distilled prior to use. Sensitive reactions were carried out in heat-dried glassware and under an argon atmosphere. Analytical thin layer chromatography (TLC) was performed on silica gel 60 F254 glass plates precoated with a 0.25 mm thickness of silica gel. Spots were visualized by staining with vanillin solution (6% (w/v) vanillin and 10% (v/v) sulfuric acid in 95% EtOH) or Hanessian's stain (5% (w/v) ammonium molybdate, 1% (w/v) cerium(II) sulfate and 10% (v/v) sulfuric acid in water). Silica column chromatography was performed on Fluka silica gel 60 (230-400 mesh).

¹H, ¹³C and two-dimensional NMR spectra were measured with a Varian 400-MR, 600-MR and Bruker Avance 700 spectrometer at 296 K. Chemical shifts (δ) are reported in parts per million (ppm) relative to the respective residual solvent peaks (CDCl₃: δ 7.27 in ¹H and 77.16 in ¹³C NMR; CD₃OD: δ 3.31 in ¹H and 49.00 in ¹³C NMR; D₂O: δ 4.80 in ¹H NMR; acetone-d₆: δ 2.05 in ¹H and 29.92 in ¹³C NMR). The following abbreviations are used to indicate peak multiplicities: s singlet; d doublet; *dd* doublet of doublets; *t* triplet; *dt* doublet of triplets; q quartet; m multiplet. Coupling constants (J) are reported in Hertz (Hz). Optical rotation (OR) measurements were carried out with a Schmidt & Haensch UniPol L1000 polarimeter at λ = 589 nm and a concentration (c) expressed in g/100 mL in the solvent noted in parentheses. High resolution mass spectrometry (HRMS) was performed at the Free University Berlin, Mass Spectrometry Core Facility, with an Agilent 6210 TOF mass spectrometer. Infrared (IR) spectra were measured with a Perkin Elmer 100 FTIR spectrometer.

### A. Synthesis of monosaccharide building blocks

### Example A.1 Synthesis of the C3-NAP protected glucose 19

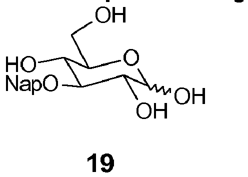

a) Diacetone glucose **17** (16.2 g, 62.2 mmol) was dissolved in anhydrous dimethylformamide (80 mL) at 0 °C. To this solution, sodium hydride (1.17 g, 74.7 mmol, 60% in mineral oil) was added and the solution was stirred until all gas evolution has ceased (2 h). To this solution, 2-(bromomethyl)naphthalene (40.8 g, 184 mmol) was added and the reaction was stirred for 16 h under argon. The reaction was quenched by slow addition of sat. aq. NH₄Cl solution. The mixture was extracted with diethyl ether and the organic layer was washed with brine. The organic layer was dried over MgSO₄ and concentrated *in vacuo* to give a viscous oil.
b) Water (0.5 L) and Amberlite^{®} IR120 (50 g, H⁺) were added to the viscous oil and the reaction was stirred at 80 °C for 16 h. After cooling to room temperature, Amberlite^{®} IR120 was filtered off and the aqueous layer was washed with a mixture of Et₂O-EtOAc 1:1. The aqueous layer was evaporated to yield a white paste, which was azeotropically dried using toluene. The pure compound 3-O-(2-naphthylmethyl)-D-glucopyranose **19** was obtained as a white solid (13.3 g, 67% over two steps). **¹H NMR** (400 MHz, CD₃OD): δ 7.89 - 7.77 (m, 4H), 7.55 (d, *J* = 8.8 Hz, 1 H), 7.44 - 7.39 (m, 2H), 5.05-4.98 (m, 2H), 4.48 (d, *J* = 7.8 Hz, 1 H), 3.83 (dd, *J* = 11.9, 1.9 Hz, 1 H), 3.63 (dd, *J* = 11.8, 5.9 Hz, 1 H), 3.41 (dt, *J* = 23.8, 8.8 Hz, 2H), 3.30-3.25 (m, 2H).

### Example A.2 Synthesis of the C3-NAP protected peracetylated glucose 20

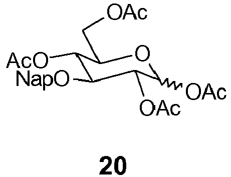

Monosaccharide **19** (16.3 g, 50.9 mmol) and sodium acetate (8.35 g, 102 mmol) were combined in stirred acetic anhydride (96 mL) and heated to 60 °C for 2 h. The reaction mixture was poured into water, forming a white precipitate. The white precipitate was dissolved in dichloromethane and washed with sat. aq. NaHCO₃ solution and brine. The organic layer was dried over MgSO₄ and the solution was concentrated *in vacuo* yielding 1,2,4,6-tetra-O-acetyl-3-O-(2-naphthylmethyl)-D-glucopyranose **20** as a pure white solid (24.1 g, 97%, α:β = 2:9). **¹H NMR** (400 MHz, CDCl₃): δ 7.80 (dd, *J* = 8.7, 3.2 Hz, 3H), 7.67 (s, 1 H), 7.52 - 7.40 (m, 2H), 7.32 (d, *J* = 8.2 Hz, 1 H), 5.63 (d, *J* = 8.2 Hz, 1 H), 5.18 (td, *J =* 9.3, 5.0 Hz, 2H), 4.76 (s, 2H), 4.21 (dd, *J* = 12.5, 4.8 Hz, 1 H), 4.07 (dd, *J* = 12.4, 2.1 Hz, 1 H), 3.78 (t, *J* = 9.3 Hz, 1 H), 3.74 - 3.69 (m, 1 H), 2.09 (s, 3H), 2.07 (s, 3H), 1.92 - 1.90 (bs, 6H). **¹³C NMR** (100 MHz, CDCl₃): δ 170.7, 169.3, 169.2, 169.1, 134.9, 133.1, 133.0, 128.3, 127.9, 127.6, 126.5, 126.3, 126.1, 125.6, 91.9, 79.9, 74.3, 73.0, 71.5, 69.0, 61.7, 20.9, 20.8, 20.7, 20.7.

### Example A.3 Synthesis of monosaccharide 21 a

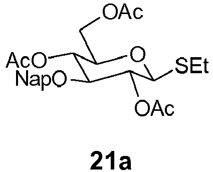

Monosaccharide **20** (5.45 g, 11.16 mmol), ethanethiol (1.45 g, 20.08 mmol) and dried acid-washed 4 Ǻ molecular sieve pellets were added to anhydrous dichloromethane (35 mL) and stirred under argon for 1 h. The solution was cooled to 0 °C, followed by drop-wise addition of boron trifluoride diethyl etherate (2.83 mL, 22.31 mmol) over 40 min. The reaction mixture was stirred for 2 h, followed by dilution with dichloromethane. The organic layer was washed with sat. aq. NaHCO₃ solution, water and brine. The organic layer was dried over MgSO₄. Concentration *in vacuo* revealed the product ethyl 2,4,6-tri-*O*-acetyl-3-*O-*(2-naphthylmethyl)-1-thio-β-D-glucopyranoside **21a** as a white foam (5.37 g, 98%). **¹H NMR** (400 MHz, CDCl₃): δ 7.84 - 7.76 (m, 3H), 7.67 (s, 1 H), 7.51 - 7.43 (m, 2H), 7.32 (d, *J* = 7.9 Hz, 1 H), 5.11 (ABq, *J* = 9.8 Hz, 2H), 4.78 - 4.72 (m, 2H), 4.38 (d, *J* = 10 Hz, 1 H), 4.19 - 4.08 (m, 2H), 3.74 (t, *J* = 9.3 Hz, 1 H), 3.62-3.55 (m, 1 H), 2.75 - 2.61 (m, 2H), 2.06 (s, 3H), 1.96 (s, 3H), 1.91 (s, 3H), 1.24 (t, *J* = 7.4 Hz, 3H).

### Example A.4 Synthesis of monosaccharide 14a

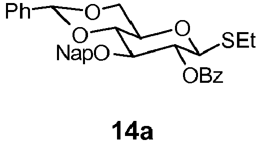

a) Monosaccharide **21a** (5.37 g, 10.90 mmol) and sodium methoxide (1.77 g, 32.7 mmol) were dissolved in anhydrous methanol (30 mL) and stirred under argon for 16 h. The resulting solution was filtered through Amberlite^{®} IR120 (3 g, H⁺). Concentration *in vacuo* revealed the product ethyl 3-*O*-(2-naphthylmethyl)-1-thio-β-D-glucopyranoside **22a** as a pure white solid.
b) This white solid was added to a stirred solution of benzaldehyde (11 mL). The addition of trifluoroacetic acid (0.52 mL, 7.03 mmol) resulted in the formation of a white precipitate after 10 min. After 1 h, the reaction was quenched by addition of triethylamine (1.5 mL). Filtration, followed by trituration with cold diethyl ether gave the product ethyl 4,6-*O*-benzylidene-3-*O*-(2-naphthylmethyl)-1-thio-β-D-glucopyranoside **23a** as a white solid.
c) Subsequently, the white solid **23a** was transferred to a stirred solution of pyridine (2 mL) in dichloromethane (20 mL) under argon. Benzoyl chloride (0.91 mL, 7.79 mmol) was added and the reaction mixture was stirred for 1 h. The reaction was quenched with methanol (3 mL), followed by dilution with dichloromethane. The organic layer was washed with sat. aq. NaHCO₃ solution, water and brine. The organic layer was dried over MgSO₄. The solution was concentrated *in vacuo* yielding title compound ethyl 2-O-benzoyl-4,6-O-benzylidene-3-O-(2-naphthylmethyl)-1-thio-β-D-glucopyranoside **14a** as a pure white solid (2.54 g, 44% over 3 steps). **¹H NMR** (400 MHz, CDCl₃): δ 7.93 (d, *J* = 7.2 Hz, 2H), 7.68 (d, *J* = 7.6 Hz, 1 H), 7.69 - 7.21 (m, 14H), 5.64 (s, 1 H), 5.36 (dd, *J* = 8.5, 1.5 Hz, 1 H), 4.97 (d, *J* = 12.2 Hz, 1 H), 4.85 (d, *J* = 12.2 Hz, 1 H), 4.59 (d, *J* = 10.0 Hz, 1H), 4.41 (dd, *J* = 10.5, 4.9 Hz, 1 H), 3.96 - 3.81 (m, 3H), 3.61-3.51 (m, 1 H), 2.75 - 2.66 (m, 2H), 1.21 (t, *J* = 7.4 Hz, 2H). **¹³C NMR** (100 MHz, CDCl₃): δ 165.2, 137.2, 135.3, 133.2, 133.1, 132.9, 129.9, 129.7, 129.1, 128.4, 128.1, 127.9, 127.6, 127.0, 126.2, 126.1, 125.9, 125.8, 101.4, 84.3, 81.8, 79.0, 74.2, 71.8, 70.7, 68.7, 24.1, 14.8. **HRMS** (ESI⁺) calculated for C₃₃H₃₂O₆Na [M + Na]⁺ 579.1817, found 579.1788.

### Example A.5 Synthesis of monosaccharide 10a

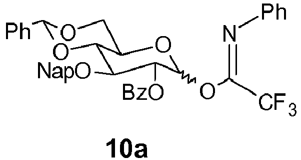

a) Monosaccharide **14a** (1.5 g, 2.63 mmol) and NIS (591 mg, 2.63 mmol) were added to a stirred solution of DCM (30 mL) and H₂O (6 mL) under nitrogen. To this solution was added trifluoromethanesulfonic acid (26 µL, 0.26 mmol) at 0 °C. After 3 h, the reaction was quenched by addition of sat. NaHCO₃ solution and the product was extracted with DCM and washed with sat. Na₂S₂O₃ solution. After phase separation, the organic phase was dried over MgSO₄ and concentrated in vacuum. Purification was achieved by silica gel flash chromatography using ethyl acetate in hexanes (2:5). This gave the product 1-*O*-benzoyl-4,6-*O*-benzylidene-3-*O*-(2-naphthylmethyl)-α-D-glucopyranose **13a** as an off-white solid.
b) Compound **13a** was dissolved in DCM (40 mL), triethylamine (7.0 mL) was added and the reaction mixture was stirred under nitrogen atmosphere overnight. The reaction mixture was concentrated in vacuum and then purified by silica gel flash chromatography using ethyl acetate in hexanes (2:5) to yield -*O*-benzoyl-4,6-*O*-benzylidene-3-*O*-(2-naphthylmethyl)-D-glucopyranose **12a** as a pure white solid (1.21 g, 89%).
c) To a stirred solution of compound **12a** (1.2 g, 2.34 mmol) in DCM (30 ml), cesium carbonate (1.52 g, 4.68 mmol) and 2,2,2-trifluoro-N-phenylacetimidoyl chloride (0.76 mL, 4.68 mmol) were added. The reaction mixture was stirred overnight under nitrogen. The reaction mixture was filtered through Celite^{®} and the filtrate was concentrated in vacuum. The crude product was purified by trituration using diethyl ether to yield title compound 2-*O*-benzoyl-4,6-*O-*benzylidene-3-*O*-(2-naphthylmethyl)-D-glucopyranosyl 2,2,2-trifluoro-N-phenyl-acetimidate **10a** as a pure white solid (1.1 g, 69%). **¹H NMR** (400 MHz, CDCl₃) δ 8.03 - 7.00 (m, 22H), 6.67 (d, *J* = 7.7 Hz, 2H), 5.65 (s, 1 H), 5.55 (t, *J* = 7.7 Hz, 1 H), 5.08 - 4.81 (m, 2H), 4.42 (dd, *J* = 10.3, 4.9 Hz, 1 H), 3.99 (d, *J* = 7.8 Hz, 2H), 3.87 (t, *J* = 10.2 Hz, 1H). **¹³C NMR** (101 MHz, CDCl₃) δ 164.9, 143.1, 137.2, 135.2, 133.5, 133.2, 133.1, 130.0, 129.4, 129.3, 128.9, 128.6, 128.5, 128.3, 128.0, 127.8, 127.1, 126.2, 126.1, 126.0, 124.6, 119.4, 101.7, 81.3, 77.7, 74.1, 72.5, 68.7,67.1.

### Synthesis of monosaccharide 10a on a larger scale:

a) Monosaccharide **14a** (10 g, 18 mmol) and NIS (3.94 g, 17.5 mmol) were added to a stirred solution of DCM (80 mL) and H₂O (4 mL) under nitrogen. To this solution was added trifluoromethanesulfonic acid (310 µL, 3.50 mmol) at 0 °C. After 5 h, the reaction was quenched by addition of sat. NaHCO₃ solution. The product was extracted with DCM and washed with sat. Na₂S₂O₃ solution. After phase separation, the organic phase was dried over MgSO₄ and concentrated in vacuum. Purification was achieved by silica gel flash chromatography using ethyl acetate in hexanes (2:5). This gave the product 1-*O*-benzoyl-4,6-*O*-benzylidene-3-*O*-(2-naphthylmethyl)-α-D-glucopyranose **13a** as an off-white solid.
b) Compound **13a** was dissolved in DCM (40 mL), triethylamine (24 mL) was added and the reaction mixture was stirred under nitrogen atmosphere overnight. The reaction mixture was concentrated in vacuum and then purified by silica gel flash chromatography using ethyl acetate in hexanes (2:5) to yield 2-*O*-benzoyl-4,6-*O*-benzylidene-3-*O*-(2-naphthylmethyl)-D-glucopyranose **12a** as a pure white solid (6.89 g, 76%).
c) To a stirred solution of compound **12a** (3.5 g, 6.83 mmol) in DCM (25 ml), cesium carbonate (4.45 g, 13.66 mmol) and 2,2,2-trifluoro-*N*-phenylacetimidoyl chloride (3.54 g, 17.07 mmol) were added. The reaction mixture was stirred overnight under nitrogen. The reaction mixture was filtered through Celite^{®} and the filtrate was concentrated in vacuum. The crude product was purified by trituration using diethyl ether to yield title compound 2-*O*-benzoyl-4,6-*O-*benzylidene-3-*O*-(2-naphthylmethyl)-D-glucopyranosyl 2,2,2-trifluoro-N-phenyl-acetimidate **10a** as a pure white solid (3.9 g, 84%).

The synthesis of monosaccharide **12a** (step a) and step b)) as well as the synthesis of monosaccharide **10a** were further carried out on different scales:

**step a) + step b)**

| **14a [g]** | **12a [g]** | **yield** |
|---|---|---|
| 0.4 g | 0.31 g | 84% |
| 0.55 g | 0.45 g | 89% |
| 1.5 g | 1.21 g | 89% |
| 5 g | 3.75 g | 83% |
| 15 g | 10.4 g | 76% |

**step c)**

| **12a [g]** | **10a [g]** | **yield** |
|---|---|---|
| 0.45 g | 0.6 g | >95%^{a} |
| 1.2 g | 1.13 g | 69% |
| 2.0 g | 2.25 g | 80% |
| 2.76 g | 3.25 g | 83% |
| 3.0 g | 3.0 g | 75% |
| 3.5 g | 3.9 g | 84% |
| 3.75 g | 4.3 g | 88% |
| 12.5 g | 7.21 g | 43% |

| | | |
|---|---|---|
| ^{a}crude product, only filtration | | |

### Example A.6 Synthesis of monosaccharide

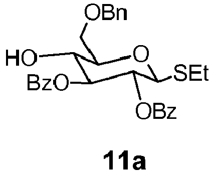

Ethyl 2,3-di-*O*-benzoyl-4,6-*O*-benzylidene-1-thio-β-D-glucopyranoside **24a** (4 g, 7.7 mmol) was dissolved in DCM (50 mL) at 0 °C. 4A molecular sieves were added and the resulting mixture was stirred for 10 min. Then triethylsilane (8.4 mL, 52.4 mmol) and trifluoroacetic acid (3.8 mL, 52.4 mmol) were added and the reaction mixture was stirred at room temperature for 16 h. The reaction was quenched by addition of triethylamine at 0 °C, filtered and concentrated in vacuum. The residue was purified by silica gel chromatography using ethyl acetate in n-hexane (1:3 to 1:1) affording the title compound ethyl 2,3-di-*O*-benzoyl-6-*O-*benzyl-1-thio-β-D-glucopyranoside **11a** as foamy gum (3.53 g, 88%). **¹H NMR** (400 MHz, CDCl₃) δ 8.10 - 7.79 (m, 4H), 7.55 - 7.15 (m, 11 H), 5.56 - 5.31 (m, 2H), 4.76 - 4.68 (m, 1 H), 4.68 - 4.57 (m, 2H), 3.98 (td, *J* = 9.3, 3.3 Hz, 1 H), 3.93 - 3.80 (m, 2H), 3.72 (ddd, *J* = 9.3, 4.9, 4.2 Hz, 1 H), 3.23 (d, *J* = 3.4 Hz, 1 H), 2.83 - 2.64 (m, 2H), 1.27 (t, *J* = 7.5 Hz, 3H). **¹³C NMR** (101 MHz, CDCl₃) δ 167.2, 165.5, 137.8, 133.5, 133.3, 130.1 (2C), 130.0 (2C), 129.5, 129.2, 128.6, 128.5 (2C), 128.4, 128.0, 127.9, 83.1, 78.9 (2C), 73.9, 71.2, 70.4, 70.3, 24.3, 15.1.

### Synthesis of monosaccharide 11a on a larger scale:

Ethyl 2,3-di-*O*-benzoyl-4,6-*O*-benzylidene-1-thio-β-D-glucopyranoside **24a** (15 g, 28.9 mmol) was dissolved in DCM (120 mL) at 0 °C. 4A molecular sieves were added and the resulting mixture was stirred for 10 min. Then triethylsilane (32 mL, 202 mmol) and trifluoroacetic acid (15 mL, 202 mmol) were added and the reaction mixture was stirred at room temperature for 12 h. The reaction was quenched by addition of sat. aq. NaHCO₃ solution (100 mL) and extracted with dichloromethane (3 x 50 mL). The organic layer was washed with brine (150 mL), dried over. MgSO₄ and concentrated *in vacuo.* The residue was purified by silica gel chromatography using ethyl acetate in n-hexanes (1:10 to 1:1) to afford the title compound ethyl 2,3-di-*O*-benzoyl-6-*O*-benzyl-1-thio-β-D-glucopyranoside **11a** as foamy gum (11.16 g, 74%).

The synthesis of monosaccharide **11a** was further carried out on different scales:

| **24a [g]** | **11a [g]** | **yield** |
|---|---|---|
| 1.65 g | 1.40 g | 85% |
| 4.0 g | 3.53 g | 89% |
| 5.0 g | 3.6 g | 72% |
| 10 g | 8.66 g | 86% |
| 15 g | 11.14 g | 74% |

### B. Synthesis of ST3 tetrasaccharide bearing an aminoethyl linker

### Example B.1 Synthesis of disaccharide 2a

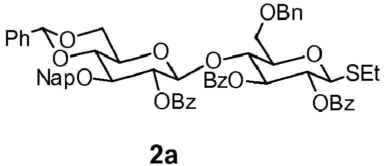

Monosaccharide **10a** (0.85 g, 1.24 mmol) and monosaccharide **11a** (0.78 g, 1.49 mmol) were dissolved in DCM (12 mL) before activated 4A acid-washed molecular sieves were added. The resulting suspension was stirred for 30 min before cooling to -10 °C. TMSOTf (34 µL, 0.186 mmol) was added and the suspension was stirred for 1 h at -10 °C. The solution was allowed to warm to 0 °C for 30 min before the reaction was quenched by addition of Et₃N (0.5 mL). The resulting mixture was filtered and concentrated *in vacuo.* Precipitation of the pure product was achieved by addition of cold diethyl ether. Additional trituration with diethyl ether gave the title compound ethyl 2,3-di-*O*-benzoyl-4-*O*-(2-*O-*benzoyl-4,6-*O*-benzylidene-3-*O*-(2-naphthylmethyl)-β-D-glucopyranosyl)-6-*O-*benzyl-1-thio-β-D-glucopyranoside **2a** as a pure white solid (950 mg, 75%). **¹H NMR** (400 MHz, CDCl₃) δ 7.98 (d, *J* = 7.1 Hz, 2H), 7.93 - 7.90 (m, 2H), 7.81 (d, *J* = 7.1 Hz, 2H), 7.68 (d, *J* = 7.8 Hz, 1 H), 7.58 - 7.31 (m, 20H), 7.22 - 7.09 (m, 5H), 5.61 (t, *J* = 9.3 Hz, 1 H), 5.38 (t, *J* = 9.7 Hz, 1 H), 5.29 (s, 1 H), 5.17 (dd, *J =* 9.0, 8.1 Hz, 1 H), 4.88 (d, *J* = 12.4 Hz, 1 H), 4.75 (d, *J* = 12.4 Hz, 1 H), 4.57 - 4.50 (m, 3H), 4.25 (d, *J* = 12.2 Hz, 1 H), 4.13 (t, *J* = 9.3 Hz, 1 H), 3.65 (dt, *J* = 8.0, 7.1 Hz, 2H), 3.58 - 3.50 (m, 2H), 3.45 (d, *J* = 9.8 Hz, 2H), 3.17 - 3.05 (m, 1 H), 2.75 - 2.60 (m, 3H), 1.20 (t, *J* = 7.5 Hz, 2H). **¹³C NMR** (101 MHz, CDCl₃) δ 165.3, 165.1, 164.6, 138.0, 137.2, 135.3, 133.2, 133.1, 133.0, 132.9, 130.2, 129.8, 129.7, 129.5, 129.3, 129.0, 128.4, 128.3 (2C), 127.9, 127.8 (2C), 127.6, 126.9, 126.1, 126.0, 125.9, 125.7, 101.2, 101.1, 83.4, 81.4, 78.7, 77.5, 75.6, 74.6, 73.7, 73.4 (2C), 70.6, 67.9, 67.4, 65.8, 24.1, 14.8. **HRMS** (ESI⁺) calculated for [M + H]⁺ 1017.3520, found 1017.3514.

### Synthesis of monosaccharide 2a on a larger scale:

Monosaccharide **10a** (1.96 g, 2.87 mmol) and monosaccharide **11a** (1.0 g, 1.91 mmol) were dissolved in DCM (12 mL) before activated 4A acid-washed molecular sieves were added. The resulting suspension was stirred for 30 min before cooling to -30 °C. TMSOTf (69 µL, 0.383 mmol) was added and the solution was stirred for 1 h at -20 °C. The mixture was allowed to warm to 0 °C for 30 min, after which the reaction was quenched by addition of Et₃N (0.5 mL). The resulting solution was filtered and concentrated in vacuum. Purification was achieved by flash chromatography using ethyl acetate in hexanes (1:4 to 1:1), which gave the title compound ethyl 2,3-di-*O*-benzoyl-4-*O*-(2-*O*-benzoyl-4,6-*O-*benzylidene-3-*O*-(2-naphthylmethyl)-β-D-glucopyranosyl)-6-*O*-benzyl-1-thio-β-D-glucopyranoside **2a** as a white solid (570 mg, 31%).

The synthesis of disaccharide **2a** was further carried out on different scales and different conditions:

| **10a [g]** | **2a [g]** | **yield** | **activator/catalyst** | **solvent** | **purification** |
|---|---|---|---|---|---|
| 0.60 g | 0.54 g | 61% | TMSOTf | DCM | trituration |
| 0.80 g | 0.785 g | 66% | TMSOTf | DCM | trituration |
| 0.85 g | 0.95 g | 75% | TMSOTf | DCM | trituration |
| 0.47 g | 0.512 g | 73% | TMSOTf | DCM | trituration and cc^{a} |
| 1.0 g | 0.57 g | 31% | TMSOTf | DCM | cc |
| 0.05 g | 0.041 g | 45% | TMSOTf | DCM/toluene | cc |
| 0.05 g | 0.047 g | 52% | TMSOTf | toluene | trituration |
| 0.25 g | 0.29 g | 64% | TfOH | DCM | trituration |
| 0.25 g | 0.32 g | 71% | TMSOTf | DCM | trituration |
| 2.8 g | 4.2 g | 82% | TMSOTf | toluene | trituration |

| | | | | | |
|---|---|---|---|---|---|
| ^{a}cc: column chromatography | | | | | |

### Example B.2 Synthesis of disaccharide 9a

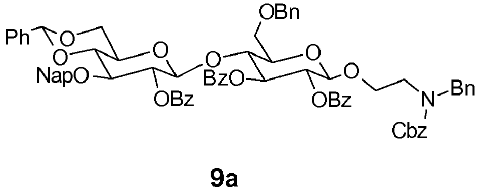

Disaccharide **2a** (150 mg, 0.147 mmol) and 2-(benzyl(benzyloxycarbonyl)amino)-ethanol (50.5 mg, 0.177 mmol) were dried azeotropically using toluene. DCM (5 mL) was added, followed by addition of activated 4A acid-washed molecular sieves and the solution was stirred at room temperature for 30 min before cooling to -10 °C. NIS (40 mg, 0.177 mmol) and TfOH (1.3 µL, 0.015 mmol) were added and the reaction mixture was stirred between -10 °C to 0 °C for 1.5 h. The reaction mixture was quenched by addition of Et₃N (50 µL), followed by addition of 10% aq. Na₂S₂O₃ solution (10 mL). The product was extracted with DCM (3 x 10 mL) and the combined organic layers were washed with brine (10 mL). The resulting organic layer was dried over MgSO₄ and concentrated *in vacuo*. Purification was achieved by flash chromatography using ethyl acetate in hexanes (1:4 to 1:2), which gave the product 2-(benzyl(benzyloxycarbonyl)amino)-ethyl 2,3-di-*O*-benzoyl-4-*O*-(2-*O*-benzoyl-3-*O*-(2-naphthylmethyl)-4,6-*O*-benzylidene-β-D-glucopyranosyl)-6-*O*-benzyl-β-D-glucopyranoside **9a** as a white solid (163 mg, 89%). **[α]_{D}²⁰** = 16.3° (c = 2, CHCl₃). **IR** (thin film, cm⁻¹): vₘₐₓ : 3033, 2875, 1733, 1700, 1454, 1267, 1094, 1072. **¹H NMR** (400 MHz, CDCl₃) δ 7.86 (dd, *J* = 7.6, 5.0 Hz, 2H), 7.76 (dd, *J* = 7.8, 1.6 Hz, 1 H), 7.66 - 7.39 (m, 5H), 7.53 - 7.38 (m, 12H), 7.37 - 7.21 (m, 20H), 7.14 - 7.12 (m, 2H), 5.62 (s, 1 H), 5.31 (dd, *J* = 9.1, 8.0 Hz, 1 H), 5.17 (d, *J* = 8.0 Hz, 2H), 5.00 (dd, *J* = 11.6, 5.0 Hz, 2H), 4.90 - 4.80 (m, 3H), 4.77 - 4.51 (m, 5H), 4.30 - 4.14 (m, 3H), 4.01 - 3.96 (m, 1 H), 3.80 (dt, *J* = 36.0, 9.2 Hz, 3H), 3.64 - 3.27 (m, 9H), 3.08 (dd, *J* = 49.9, 9.8 Hz, 1 H) **¹³C NMR** (101 MHz, CDCl₃) δ 164.9, 156.6, 156.3, 139.2, 138.6, 138.5, 138.1, 138.0, 137.9, 137.4, 136.7, 136.6, 135.5, 133.4, 133.2, 133.0, 129.9, 129.6, 129.2, 128.6, 128.5 (2C), 128.4, 128.3, 128.1 (2C), 128.0 (2C), 127.9 (2C), 127.7 (2C), 127.5, 127.3, 127.0, 126.2, 126.0, 125.9, 103.7, 101.4, 100.8, 82.8, 82.1, 81.8, 77.7, 76.6, 75.5, 75.0, 74.9, 74.4, 74.3, 74.0, 73.6, 68.8, 68.0, 67.6, 67.4, 67.3, 66.1, 51.6, 47.2, 46.1. **HRMS** (ESI⁺) calculated for C₇₅H₇₃NO₁₄Na [M + Na]⁺ 1234.4929, found 1234.4957.

The synthesis of disaccharide **9a** was also performed on a larger scale. When 250 mg of disaccharide **2a** were employed according to the procedure described above, 243 mg of disaccharide **9a** were obtained (80% yield).

### Example B.3 Synthesis of disaccharide 3a

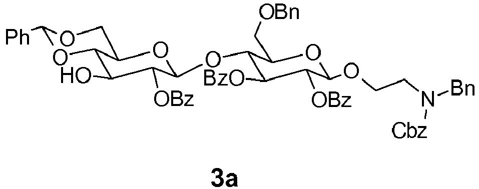

Disaccharide **9a** (155 mg, 0.13 mmol) was transferred to a stirred mixture of dichloromethane (7 mL) and water (135 µL), and subsequently cooled (5 to 10 °C) for 5 min. DDQ (128 mg, 0.56 mmol) was added slowly over a period of 2.5 h, until all of the starting disaccharide **9a** had been consumed (observed by TLC analysis). The reaction was quenched by addition of sat. aq. NaHCO₃ (5 mL) and extracted with dichloromethane (2 x 50 mL). The organic layer was dried over MgSO₄ and concentrated *in vacuo.* Purification was achieved by flash chromatography using ethyl acetate in hexanes (1:4 to 1:1), which gave the product 2-(benzyl(benzyloxycarbonyl)amino)-ethyl 2,3-di-*O*-benzoyl-4-*O*-(2-*O-*benzoyl-4,6-*O*-benzylidene-β-D-glucopyranosyl)-6-*O*-benzyl-β-D-glucopyranoside 3a as a white solid (115 mg, 84%). **[α]_{D}²⁰** = -6.7° (c = 1, CHCl₃). **IR** (thin film, cm⁻¹): vₘₐₓ : 3424, 3033, 2881, 1732, 1700, 1455, 1269, 1094, 1056, 1029. **¹H NMR** (400 MHz, CDCl₃) δ 7.99 (t, *J* = 7.3 Hz, 2H), 7.61 (t, *J* = 7.5 Hz, 1 H), 7.52 - 7.44 (m, 4H), 7.43 - 7.17 (m, 27H), 7.11 - 7.07 (m, 1 H), 5.48 (s, 1 H), 5.18 - 5.12 (m, 3H), 4.93 (d, *J =* 10.8 Hz, 1 H), 4.84 - 4.75 (m, 3H), 4.72 - 4.63 (m, 2H), 4.59 - 4.48 (m, 2H), 4.32 (d, *J =* 12.1 Hz, 1 H), 4.27 - 4.11 (m, 2H), 4.03 - 3.78 (m, 3H), 3.73 - 3.32 (m, 9H), 3.27 (td, *J* = 9.7, 5.0 Hz, 1 H), 3.10 (dd, *J* = 47.0, 9.8 Hz, 1 H), 2.58 (br s, 1 H). **¹³C NMR** (101 MHz, CDCl₃) δ 165.6, 156.6, 156.3, 139.2, 138.6, 138.5, 138.2, 138.0, 137.9, 137.0, 136.7, 136.6, 133.6, 130.0, 129.5, 128.7, 128.6, 128.5, 128.4, 128.3, 128.1 (2C), 128.0, 127.9, 127.6 (2C), 127.5, 127.3, 126.4, 103.7, 102.0, 100.7, 82.8, 81.8, 81.1, 76.8, 75.5, 75.1, 75.0 (2C), 74.5, 74.4, 73.6, 72.6, 68.7, 68.0, 67.9, 67.5, 67.4, 66.2, 51.6, 47.2, 46.1. **HRMS** (ESI⁺) calculated for C₆₄H₆₅NO₁₄Na [M + Na]⁺ 1094.4303, found 1094.4311.

### Synthesis of disaccharide 3a on a larger scale:

Disaccharide **9a** (3.0 g, 2.42 mmol) was transferred to a stirred mixture of dichloromethane (100 mL) and water (2 mL), and subsequently cooled (5 to 10 °C) for 5 min. DDQ (2.47 g, 10.88 mmol) was added slowly over a period of 2.5 h, until all of the starting disaccharide **9a** had been consumed (observed by TLC analysis). The reaction was quenched by addition of sat. aq. NaHCO₃ (40 mL) and extracted with dichloromethane (2 x 25 mL). The organic layer was dried over Na₂SO₄ and concentrated *in vacuo.* Purification was achieved by flash chromatography using ethyl acetate in hexanes (1:4 to 1:1), which gave the product 2-(benzyl(benzyloxycarbonyl)amino)-ethyl 2,3-di-*O*-benzoyl-4-*O*-(2-*O-*benzoyl-4,6-*O*-benzylidene-β-D-glucopyranosyl)-6-*O*-benzyl-β-D-glucopyranoside **3a** as a white solid (2.1 g, 79%).

The synthesis of disaccharide **3a** was further carried out on different scales:

| **9a [g]** | **3a [g]** | **yield** |
|---|---|---|
| 0.155 g | 0.155 g | 84% |
| 0.161 g | 0.103 g | 72% |
| 0.25 g | 0.12 g | 55% |
| 3.0 g | 2.1 g | 79% |

### Example B.4 Synthesis of tetrasaccharide 1a

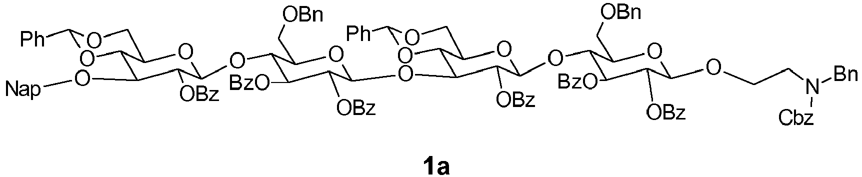

Disaccharide **3a** (110 mg, 0.10 mmol) and disaccharide **2a** (132 mg, 0.13 mmol) were dried azeotropically using toluene. DCM (4 mL) and 4A molecular sieves were added. The solution was stirred at room temperature for 30 min and subsequently cooled to -10 °C. NIS (30 mg, 0.135 mmol) and TfOH (0.9 µL, 0.01 mmol) were added and the reaction mixture was stirred between -10 °C to 0 °C for 1.5 h. The reaction was quenched by addition of sat. aq. NaHCO₃ solution (1 mL) and filtered. To the filtrate was added 10% aq. Na₂S₂O₃ solution (20 mL) and the resulting solution was diluted with DCM, followed by extraction with DCM (3 x 15 mL). The combined organic phases were washed with sat. aq. NaHCO₃ solution (15 mL) and brine (10 mL). The organic layer was dried over MgSO₄ and concentrated *in vacuo.* Purification was achieved by flash chromatography using ethyl acetate in hexanes (1:4 to 1:1), which gave the product 2-(benzyl(benzyloxycarbonyl)amino)ethyl 2-*O*-benzoyl-4,6-*O*-benzylidene-3-*O*-(2-naphthylmethyl)-β-D-glucopyranosyl-(1→4)-2,3-di-*O*-benzoyl-6-*O*-benzyl-β-D-glucopyranosyl-(1→3)-2-*O*-benzoyl-4,6-*O*-benzylidene-β-D-glucopyranosyl-(1→4)-2,3-di-*O*-benzoyl-6-*O*-benzyl-β-D-glucopyranoside **1a** as a white solid (139 mg, 68%). **¹H NMR** (400 MHz, CDCl₃) δ 8.00 (d, *J* = 7.7 Hz, 1 H), 7.97 - 7.82 (m, 3H), 7.70 (d, *J* = 7.7 Hz, 1 H), 7.67 - 7.59 (m, 1 H), 7.58 - 6.84 (m, 26H), 5.59 - 5.42 (m, 1 H), 5.41 - 5.31 (m, 1 H), 5.26 (s, 1 H), 5.19 (d, *J* = 1.4 Hz, 1 H), 5.12 (td, *J* = 14.9, 8.0 Hz, 1 H), 5.02 (d, *J* = 7.0 Hz, 1 H), 4.95 (s, 1 H), 4.87 (d, *J =* 12.4 Hz, 1 H), 4.74 (d, *J* = 12.4 Hz, 1 H), 4.60 (d, *J* = 8.0 Hz, 1 H), 4.55 (d, *J =* 11.4 Hz, 1 H), 4.47 (dd, *J* = 15.7, 7.9 Hz, 1 H), 4.42 - 4.32 (m, 1 H), 4.32 - 4.27 (m, 1 H), 4.24 (d, *J* = 12.1 Hz, 1 H), 4.15 - 4.02 (m, 1 H), 3.94 (t, *J* = 9.3 Hz, 1 H), 3.80 (t, *J* = 8.4 Hz, 1 H), 3.71 - 3.58 (m, 1 H), 3.56 - 3.36 (m, 2H), 3.33 - 3.18 (m, 2H), 3.18 - 3.02 (m, 2H), 2.69 - 2.57 (m, 1 H). **¹³C NMR** (101 MHz, CDCl₃) δ 165.3, 165.0, 164.7, 164.5, 138.1, 137.2, 136.8, 135.2, 133.2, 133.0, 132.9, 132.5, 130.1, 129.9, 129.8, 129.8, 129.6, 129.2, 129.1, 128.5, 128.4, 128.3, 128.3, 128.1, 128.0, 127.8, 127.8, 127.7, 127.6, 127.2, 126.9, 126.2, 126.0, 126.0, 125.9, 125.7, 101.3, 101.1, 101.0, 101.0, 100.8, 100.1, 81.4, 79.3, 77.4, 77.2, 77.0, 76.7, 75.8, 75.4, 74.1, 73.7, 73.6, 73.3, 73.1, 72.1, 68.8, 67.7, 67.0, 66.1, 65.7, 51.5. **MALDI MS** (TOF): [M + K]⁺ calculated: 2092.7, found: 2093.1.

### Synthesis of tetrasaccharide 1a on a larger scale:

Disaccharide **3a** (1.0 g, 0.91 mmol) and disaccharide **2a** (1.12 g, 1.18 mmol) were dried azeotropically using toluene. DCM (25 mL) and 4A molecular sieves (2.0 g) were added. The solution was stirred at room temperature for 30 min and subsequently cooled to -10 °C. NIS (0.27 g, 1.18 mmol) and TfOH (16 µL, 0.18 mmol) were added and the reaction mixture was stirred between -10 °C to 0 °C for 1.5 h. The reaction was quenched by addition of sat. aq. NaHCO₃ solution (1 mL) and filtered. To the filtrate was added 10% aq. Na₂S₂O₃ solution (20 mL) and the resulting solution was diluted with DCM, followed by extraction with DCM (3 x 20 mL). The combined organic phases were washed with sat. aq. NaHCO₃ solution (10 mL) and brine (10 mL). The organic layer was dried over Na₂SO₄ and concentrated *in vacuo.* Purification was achieved by flash chromatography using ethyl acetate in hexanes (1:9 to 1:4), which gave the product 2-(benzyl(benzyloxycarbonyl)amino)ethyl 2-*O*-benzoyl-4,6-*O*-benzylidene-3-*O*-(2-naphthylmethyl)-β-D-glucopyranosyl-(1→4)-2,3-di-*O*-benzoyl-6-*O*-benzyl-β-D-glucopyranosyl-(1→3)-2-*O*-benzoyl-4,6-*O*-benzylidene-β-D-glucopyranosyl-(1→4)-2,3-di-*O*-benzoyl-6-*O*-benzyl-β-D-glucopyranoside **1a** as a white solid (1.2 g, 64%).

The synthesis of tetrasaccharide **1a** was also performed with 250 mg of disaccharide **3a.** 330 mg of tetrasaccharide **1a** were then obtained (70% yield).

### Example B.5 Synthesis of tetrasaccharide 4a

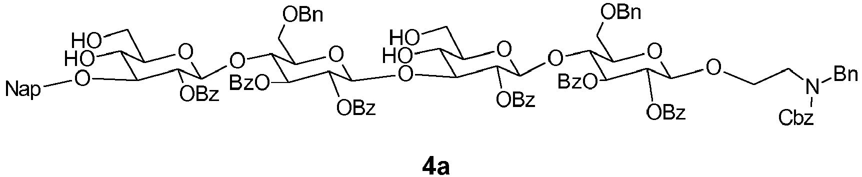

Compound **1a** (139 mg, 0.068 mmol), PTSA (1.9 mg, 0.012 mmol) and EtSH (73 µL, 1.02 mmol) were added to DCM (5 mL) and the resulting solution was stirred at room temperature for 4 h. The reaction mixture was quenched with Et₃N (50 µL) and concentrated *in vacuo.* Purification was achieved by flash chromatography using ethyl acetate in hexanes (1:5 to 100% ethyl acetate) obtaining 2-(benzyl(benzyloxycarbonyl)amino)ethyl 2-*O*-benzoyl-3-*O*-(2-naphthyl-methyl)-β-D-glucopyranosyl-(1→4)-2,3-di-*O*-benzoyl-6-*O*-benzyl-β-D-gluco-pyranosyl-(1→3)-2-*O*-benzoyl-β-D-glucopyranosyl-(1→4)-2,3-di-*O*-benzoyl-6-*O-*benzyl-β-D-glucopyranoside **4a** as white foam (120 mg, 94%). **¹H NMR** (400 MHz, CDCl₃) δ 7.97 (d, *J* = 7.5 Hz, 1 H), 7.93 - 7.81 (m, 3H), 7.77 - 7.70 (m, 1 H), 7.70 - 7.64 (m, 1 H), 7.57 (ddd, *J* = 14.0, 12.4, 7.7 Hz, 2H), 7.52 - 7.12 (m, 22H), 5.52 (t, *J* = 9.2 Hz, 1 H), 5.47 - 5.32 (m, 1 H), 5.27 (dd, *J* = 9.6, 7.8 Hz, 1 H), 5.14 (t, 1 H), 5.06 (dd, *J* = 10.5, 6.6 Hz, 1 H), 5.00 (dd, *J* = 13.5, 8.1 Hz, 1 H), 4.81 (d, *J* = 11.9 Hz, 1 H), 4.75 (d, *J* = 7.5 Hz, 1 H), 4.61 (t, *J* = 11.9 Hz, 1 H), 4.51 (d, *J =* 7.8 Hz, 1 H), 4.46 - 4.31 (m, 2H), 4.27 (dd, *J* = 12.8, 8.6 Hz, 1 H), 4.18 (d, *J =* 11.8 Hz, 1 H), 4.15 (d, *J* = 7.2 Hz, 1 H), 4.10 - 4.03 (m, 1 H), 4.00 (t, *J* = 9.3 Hz, 1 H), 3.93 - 3.85 (m, 1 H), 3.64 - 3.49 (m, 3H), 3.45 (d, *J* = 8.1 Hz, 1 H), 3.36 (dd, *J* = 14.7, 6.8 Hz, 2H), 3.29 - 3.16 (m, 2H), 3.07 - 2.96 (m, 1 H). **MALDI MS** (TOF): [M + K]⁺ calculated: 1916.6, found: 1917.0.

### Synthesis of tetrasaccharide 4a on a larger scale:

Compound **1a** (400 mg, 0.20 mmol), PTSA (18.7 mg, 0.09 mmol) and EtSH (210 µL, 2.92 mmol) were added to DCM (5 mL) and the resulting solution was stirred at room temperature for 4 h. The reaction mixture was quenched with Et₃N (500 µL) and concentrated *in vacuo.* Purification was achieved by flash chromatography using ethyl acetate in hexanes (1:5 to 100% ethyl acetate) obtaining 2-(benzyl(benzyloxycarbonyl)amino)ethyl 2-*O*-benzoyl-3-*O***-**(2-naphthyl-methyl)-β-D-glucopyranosyl-(1→4)-2,3-di-*O*-benzoyl-6-*O*-benzyl-β-D-glucopyranosyl-(1→3)-2-*O*-benzoyl-β-D-glucopyranosyl-(1→4)-2,3-di-*O*-benzoyl-6-*O-*benzyl-β-D-glucopyranoside **4a** as white foam (250 mg, 68%).

The synthesis of tetrasaccharide **4a** was further carried out on different scales:

| **1a [g]** | **4a [g]** | **yield** |
|---|---|---|
| 0.139 g | 0.120 g | 94% |
| 0.185 g | 0.127 g | 75% |
| 0.30 g | 0.23 g | 84% |
| 0.40 g^{a} | 0.25 g | 68% |

| | | |
|---|---|---|
| ^{a}crude | | |

### Example B.6 Synthesis of tetrasaccharide 5a

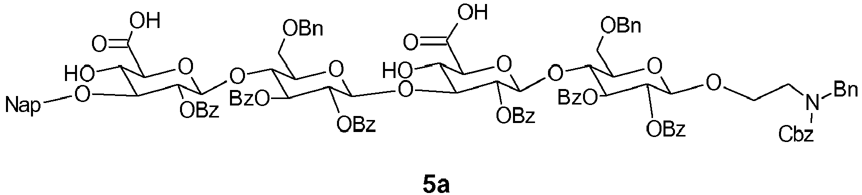

Tetrasaccharide **4a** (34 mg, 0.018 mmol) was taken in a mixture of DCM-water (0.7 mL/0.14 mL) and cooled to 0 °C. TEMPO (0.57 mg, 0.004 mmol) and BAIB (35 mg, 0.109 mmol) were added. The mixture was stirred at 0 °C for 20 min and slowly warmed to room temperature, followed by additional stirring for 4 h. The reaction mixture was then diluted with DCM (10 mL) and water (5 mL) and the aqueous layer was extracted with DCM (4 x 10 mL). The combined organic extracts were dried over MgSO₄ and concentrated *in vacuo.* The crude product was then purified by flash chromatography using DCM (100%), DCM-acetone (10:1) and finally DCM-acetone-AcOH (100:10:1) to obtain the title compound 2-(benzyl(benzyloxycarbonyl)amino)ethyl 2-*O*-benzoyl-3-*O*-(2-naphthylmethyl)-β-D-glucopyranuronosyl-(1→4)-2,3-di-*O*-benzoyl-6-*O*-benzyl-β-D-glucopyranosyl-(1→3)-2-*O*-benzoyl-β-D-glucopyranuronosyl-(1→4)-2,3-di-*O*-benzoyl-6-*O*-benzyl-β-D-glucopyranoside **5a** as an off-white solid. This compound was transferred to the next reaction without additional purification. **¹H NMR** (400 MHz, CD₃OD) δ 8.01 - 6.74 (m, 57H), 5.50 -5.36 (m, 2H), 5.18 - 5.03 (m, 2H), 5.02 - 4.83 (m, 5H), 4.71 - 4.59 (m, 3H), 4.49 - 4.45 (m, 3H), 4.41 - 4.02 (m, 7H), 3.93 - 3.35 (m, 13H), 3.26 - 3.06 (m, 3H). **MALDI MS** (TOF): [M + Na]⁺ calculated: 1929.946, found: 1929.967.

### Synthesis of tetrasaccharide 5a on a larger scale:

Tetrasaccharide **4a** (100 mg, 0.05 mmol) was taken in a mixture of DCM-water (3 mL/0.6 mL) and cooled to 0 °C. TEMPO (2 mg, 0.01 mmol) and BAIB (103 mg, 0.32 mmol) were added and the solution was stirred at 0 °C for 20 min and slowly warmed to room temperature, followed by additional stirring for 4 - 6 h. The reaction mixture was then diluted with DCM (5 mL) and water (5 mL) and the aqueous layer was extracted with DCM (3 x 15 mL). The combined organic phases were dried over Na₂SO₄ and concentrated in vacuum. The crude product was then purified by flash chromatography using DCM (100%), DCM-acetone-AcOH (9:1:0 to 9:1:0.2) to obtain the title compound 2-(benzyl(benzyloxycarbonyl)amino)ethyl 2-*O*-benzoyl-3-*O*-(2-naphthylmethyl)-β-D-glucopyranuronosyl-(1→4)-2,3-di-*O*-benzoyl-6-*O*-benzyl-β-D-glucopyranosyl-(1→3)-2-*O*-benzoyl-β-D-glucopyranuronosyl-(1→4)-2,3-di-*O*-benzoyl-6-*O*-benzyl-β-D-glucopyranoside **5a** as an off-white solid (63 mg, 62%).

The synthesis of tetrasaccharide **5a** was also performed with 145 mg of tetrasaccharide **4a.** 39 mg of tetrasaccharide **5a** were then obtained (62% yield).

### Example B.7 Synthesis of tetrasaccharide 7a

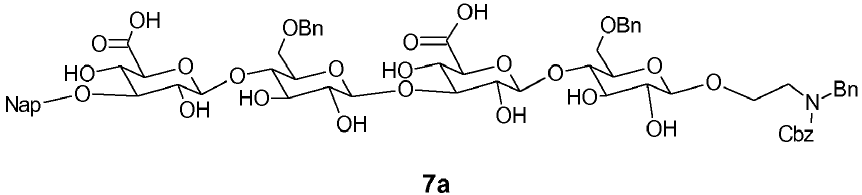

Crude compound **5a** (34.5 mg, 0.018 mmol) from the previous step and sodium methoxide (20 mg, 0.36 mmol) were dissolved in methanol (3 mL). The resulting solution was stirred for 4 d at 40 °C. The resulting solution was neutralized by addition of Amberlite^{®} IR120 H⁺ resin, followed by stirring for 10 min. The clear suspension was filtered and washed thoroughly with methanol. The filtrate was concentrated *in vacuo* and subsequently triturated with ethyl acetate in hexanes (1:10). The title compound 2-(benzyl(benzyloxycarbonyl)amino)ethyl 3-*O*-(2-naphthylmethyl)-β-D-glucopyranuronosyl-(1→4)-6-*O*-benzyl-β-D-glucopyranosyl-(1→3)-β-D-glucopyranuronosyl-(1→4)-6-*O*-benzyl-β-D-glucopyranoside **7a** was obtained as an off-white solid (20.2 mg, 87% over two steps), which was transferred to the next step without any additional purification. **¹H NMR** (400 MHz, CD₃OD) δ 8.16 - 6.99 (m, 27H), 5.11 (d, *J* = 9.2 Hz, 2H), 5.05 (s, 2H), 4.67 - 4.32 (m, 9H), 4.20 (dd, *J* = 31.8, 8.0 Hz, 1 H), 4.00 - 3.15 (m, 24H). **MALDI MS** (TOF): [M-H+Na]⁺ calculated: 1304.2898, found: 1304.764.

### Synthesis of tetrasaccharide 7a on a larger scale:

Compound **5a** (62 mg, 0.032 mmol) was taken in THF (4 mL) and 0.5 M methanolic solution of sodium methoxide (2.1 mL, 1.07 mmol) was added to it. The resulting solution was stirred at 40 °C for 16 h. The reaction mixture was evaporated in vacuum and then diluted with methanol (5 mL) and neutralized by addition of Amberlite^{®} 120 H⁺ resin. The mixture was filtered and washed thoroughly with methanol. The filtrate was concentrated in vacuum to a pale yellow solid. The crude product was subsequently triturated with diethyl ether (3 x 2 mL). The title compound 2-(benzyl(benzyloxycarbonyl)amino)ethyl 3-*O*-(2-naphthylmethyl)-β-D-glucopyranuronosyl-(1→4)-6-*O*-benzyl-β-D-glucopyranosyl-(1→)-β-D-glucopyranuronosyl-(1→4)-6-*O*-benzyl-β-D-glucopyranoside **7a** was obtained as an off-white solid (35 mg, 84%), which was transferred to the next step without any additional purification.

The synthesis of tetrasaccharide **7a** was also performed with 90 mg of tetrasaccharide **5a.** 55 mg of tetrasaccharide **7a** were then obtained (91 % yield).

### Example B.8 Synthesis of tetrasaccharide 8a

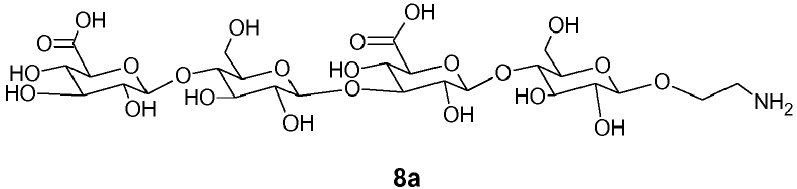

Tetrasaccharide **7a** (20 mg) was taken in a mixture of H₂O-*^{t}*BuOH-DCM (1 mL/2 mL/0.2 mL), and 20% Pd(OH)₂/C (22 mg) was added. The solution was degassed and subsequently stirred over a hydrogenous atmosphere for 24 h. The reaction mixture was then filtered through a PTFE hydrophobic filter and the filter washed thoroughly with methanol (5 x 3 mL), water-methanol (5 x 3 mL), and finally with NH₄OH in methanol (3 mL in 15 mL). After evaporation of the filtrate and drying under vacuum, the target compound 2-aminoethyl β-D-glucopyranuronosyl-(1→4)-β-D-glucopyranosyl-(1→3)-β-D-glucopyranuronosyl-(1→4)-β-D-glucopyranoside **8a** (9.2 mg, 79%) was obtained as a white material. **¹H NMR** (400 MHz, D₂O) δ 4.84 (d, *J* = 8.0 Hz, 1 H), 4.56 (d, *J* = 8.0 Hz, 2H), 4.53 (d, *J* = 7.9 Hz, 1 H), 4.14 (dt, *J* = 11.5, 4.9 Hz, 1 H), 4.04 - 3.92 (m, 3H), 3.89 - 3.75 (m, 5H), 3.72 - 3.49 (m, 10H), 3.43 - 3.34 (m, 3H), 3.29 (t, *J* = 5.1 Hz, 2H) **¹³C NMR** (101 MHz, D₂O) δ 175.4, 175.2, 102.3, 102.2, 102.0, 101.8, 82.7, 78.9, 78.6, 75.7, 75.65, 75.2, 74.8, 74.7, 74.1 (2C), 73.1, 72.9, 72.87, 72.7, 71.6, 70.1, 65.7, 60.0, 59.84, 39.3. **HRMS** (ESI⁺): calculated for C₂₆H₄₃NNaO₂₃ [M + Na]⁺, 760.2124, found 760.2134.

### Synthesis of tetrasaccharide 8a on a larger scale:

Tetrasaccharide **7a** (35 mg) was taken in a mixture of H₂O-*^{t}*BuOH-DCM (0.2 mL/1 mL/0.5 mL), and a suspension of 10% Pd/C (10 mg) in *^{t}*BuOH (1 mL) was added. The reaction mixture was purged under hydrogen gas and subsequently stirred over a hydrogenous atmosphere for 24 h at rt. The reaction mixture was then filtered through a PTFE hydrophobic filter and the filter was washed thoroughly with methanol (5 x 3 mL), water-methanol (6:4, 5 x 3 mL). The filtrate was evaporated to dryness under vacuum to get an off-white solid (17 mg, 84.5% crude yield). The crude product was then purified by C₁₈ Sepak column chromatography using water and water-methanol to get the desired major product in water fractions. Lyophilization of the water fractions yielded the desired product 2-aminoethyl β-D-glucopyranuronosyl-(1→4)-β-D-glucopyranosyl-(1→3)-β-D-glucopyranuronosyl-(1→4)-β-D-glucopyranoside **8a** as a white puffy solid (13 mg, 65%).

The synthesis of tetrasaccharide **8a** was also performed with 55 mg of tetrasaccharide **7a.** 19 mg of pure tetrasaccharide **8a** were then obtained (60% yield; 73% yield of the crude product).

### C. Synthesis of ST3 tetrasaccharide bearing an aminopentyl linker

### Example C.1 Synthesis of disaccharide 9b

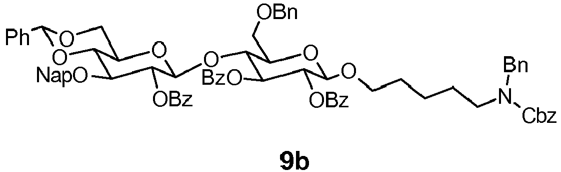

Ethyl 2,3-di-*O*-benzoyl-4-*O*-(2-*O*-benzoyl-4,6-*O*-benzylidene-3-*O*-(2-naphthyl-methyl)-β-D-glucopyranosyl)-6-*O*-benzyl-1-thio-β-D-glucopyranoside **2a** (150 mg, 0.147 mmol) and protected amino alcohol HO-(CH₂)₅-N(Bn)Cbz (62.8 mg, 0.192 mmol) were dried azeotropically using toluene. Dichloromethane (5 mL) was added, followed by the addition of activated acid-washed 4A MS and the solution was stirred at room temperature for 30 min before cooling to -10 °C. NIS (40 mg, 0.177 mmol) and TfOH (1.3 µL, 0.015 mmol) were added and the reaction mixture was stirred between -10 °C to 0 °C for 1.5 h. The reaction mixture was quenched with Et₃N (50 µL), followed by addition of 10% aq. Na₂S₂O₃ solution (10 mL). The product was extracted with DCM (3 x 10 mL) and the combined organic phases were washed with brine (10 mL). The resulting organic layer was dried over MgSO₄ and concentrated *in vacuo.* Purification was achieved by flash chromatography using ethyl acetate in hexanes (1:4 to 1:2), which gave the product 5-(benzyl(benzyloxycarbonyl)amino)-pentyl 2,3-di-*O*-benzoyl-4-*O*-(2-*O-*benzoyl-3-*O*-(2-naphthylmethyl)-4,6-*O*-benzylidene-β-D-glucopyranosyl)-6-*O-*benzyl-β-D-glucopyranoside **9b** as a white solid (189 mg, 85%). **¹H NMR** (400 MHz, CDCl₃) δ 7.98 (d, 2H), 7.86 (d, *J* = 7.6 Hz, 2H), 7.81 (d, *J* = 7.3 Hz, 2H), 7.67 (d, *J* = 7.7 Hz, 1 H), 7.58 - 7.03 (m, 35H), 5.57 (t, *J* = 9.4 Hz, 1 H), 5.34 - 5.24 (m, 2H), 5.16 (t, 1H), 5.11 (s, 2H), 4.87 (d, *J =* 12.4 Hz, 1H), 4.75 (d, *J* = 12.4 Hz, 1 H), 4.58 - 4.40 (m, 3H), 4.34 (d, *J =* 10.4 Hz, 2H), 4.23 (d, *J =* 12.3 Hz, 1 H), 4.08 (t, *J* = 9.4 Hz, 1 H), 3.72 (s, 1 H), 3.68 - 3.59 (m, 2H), 3.58 - 3.47 (m, 2H), 3.41 (d, *J* = 10.5 Hz, 2H), 3.36 - 3.23 (m, 1 H), 3.17 - 3.05 (m, 1 H), 3.05 - 2.84 (m, 2H), 2.70 (t, *J* = 10.4 Hz, 1 H), 1.45 - 1.25 (m, 4H), 1.18 - 0.95 (m, 2H). **¹³C NMR** (101 MHz, CDCl₃) δ 165.1, 164.6, 137.2, 135.3, 133.2, 133.0, 132.9, 129.8, 129.8, 129.6, 129.5, 129.0, 128.4, 128.4, 128.2, 127.9, 127.8, 127.8, 127.6, 126.9, 126.1, 126.0, 125.9, 125.7, 101.2, 101.0, 100.9, 81.4, 75.8, 74.5, 73.7, 73.4, 67.0, 65.8, 50.3, 47.0, 46.1, 29.0, 28.9, 22.9. **HRMS** (ESI⁺) calculated for C₇₈H₇₆NO₁₆Na [M + Na]⁺ 1305.5062, found 1305.5056.

### Example C.2 Synthesis of disaccharide 3b

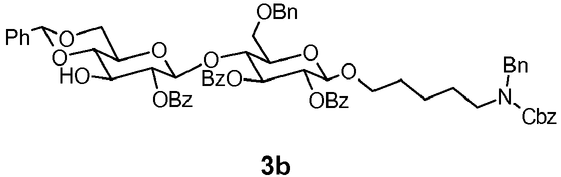

Disaccharide **9b** (145 mg, 0.113 mmol) was transferred to a stirred solution of dichloromethane (7 mL) and water (122 µL), and subsequently cooled (5 to 10 °C) for 5 min. DDQ (115 mg, 0.51 mmol) was added slowly over a period of 2.5 h, until all of the starting disaccharide **9b** had been consumed (observed by TLC analysis). The reaction was quenched with sat. aq. NaHCO₃ solution (5 mL) and extracted with dichloromethane (2 x 50 mL). The organic layer was dried over anhydrous MgSO₄ and concentrated *in vacuo.* Purification was achieved by flash chromatography using ethyl acetate in hexanes (1:4 to 1:1), which gave the product 5-(benzyl(benzyloxycarbonyl)amino)-pentyl 2,3-di-*O*-benzoyl-4-*O*-(2-*O-*benzoyl-4,6-*O*-benzylidene-β-D-glucopyranosyl)-6-*O*-benzyl-β-D-glucopyranoside **3b** as a white foam (112 mg, 87%). **¹H NMR** (400 MHz, CDCl₃) δ 7.98 (d, *J* = 8.1 Hz, 4H), 7.86 (d, *J* = 7.6 Hz, 2H), 7.65 - 7.14 (m, 29H), 5.58 (t, *J* = 9.4 Hz, 1 H), 5.31 (t, 1 H), 5.21 (s, 1 H), 5.14 - 5.01 (m, 3H), 4.66 (d, *J* = 7.9 Hz, 1 H), 4.62 (d, *J =* 12.3 Hz, 1 H), 4.54 - 4.39 (m, 1H), 4.36 (d, *J* = 12.0 Hz, 3H), 4.14 (t, *J* = 9.3 Hz, 1 H), 3.84 - 3.71 (m, 2H), 3.68 (dd, *J* = 11.1, 3.1 Hz, 1 H), 3.61 (dd, *J =* 10.6, 5.0 Hz, 1 H), 3.52 (d, *J* = 10.8 Hz, 1 H), 3.47 - 3.39 (m, 1 H), 3.31 (t, *J =* 9.3 Hz, 2H), 3.12 (td, *J* = 9.7, 5.1 Hz, 1 H), 3.03 - 2.83 (m, 2H), 2.66 (t, *J =* 10.3 Hz, 1 H), 1.39 - 1.24 (m, 4H), 1.13 - 0.98 (m, 2H). **¹³C NMR** (101 MHz, CDCl₃) δ 165.4, 165.1, 136.7, 133.4, 133.0, 129.9, 129.7, 129.6, 128.5, 128.5, 128.4, 128.3, 128.2, 127.9, 127.9, 127.8, 126.2, 101.6, 101.0, 100.9, 80.5, 75.9, 74.7, 74.6, 73.5, 71.8, 67.1, 65.8, 50.0, 45.3, 28.8, 22.8. **HRMS** (ESI⁺) calculated for C₆₇H₆₇NO₁₆Na [M + Na]⁺ 1164.4358, found 1164.4353.

### Example C.3 Synthesis of tetrasaccharide 4b

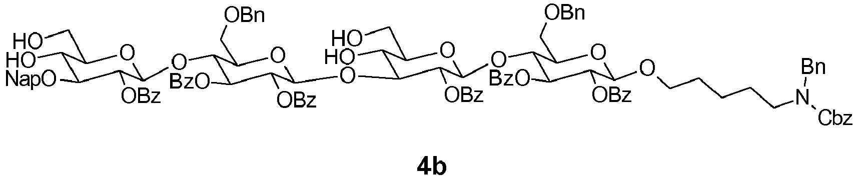

a) Disaccharide **3b** (129 mg, 0.113 mmol) and donor disaccharide **2b** (149 mg, 0.147 mmol) were dried azeotropically using toluene. Dichloromethane (3 mL) and 4A MS were added and the mixture was stirred at room temperature for 30 min and subsequently cooled to -10 °C. NIS (34 mg, 0.152 mmol) and TfOH (0.9 µL, 0.01 mmol) were added and the reaction mixture was stirred between -10 °C to 0 °C for 1.5 h. The reaction was quenched by addition of sat. aq. NaHCO₃ solution (1 mL) and filtered. To the filtrate was added 10% aq. Na₂S₂O₃ solution (20 mL) and the resulting solution was diluted with dichloromethane, followed by extraction with dichloromethane (3 x 15 mL). The combined organic phases were washed with sat. aq. NaHCO₃ solution (15 mL) and brine (10 mL). The organic layer was dried over MgSO₄ and concentrated *in vacuo*.
b) The subsequent crude solid (containing **1b**) was transferred to a stirred solution of PTSA (3.7 mg, 0.02 mmol) and EtSH (141 µL, 1.95 mmol) in dichloromethane (3.5 mL) and the resulting solution was stirred at room temperature for 3 h. The reaction mixture was quenched with Et₃N (50 µL) and concentrated *in vacuo*. Purification was achieved by flash chromatography using ethyl acetate in hexanes (1:5 to 100% ethyl acetate) obtaining the tetrasaccharide product 5-(benzyl(benzyloxycarbonyl)amino)-pentyl 2-*O*-benzoyl-3-*O*-(2-naphthylmethyl)-β-D-glucopyranosyl-(1→4)-2,3-di-*O*-benzoyl-6-*O*-benzyl-β-D-glucopyranosyl-(1→3)-2-*O*-benzoyl-β-D-glucopyranosyl-(1→4)-2,3-di-*O*-benzoyl-6-*O*-benzyl-β-D-glucopyranoside **4b** as white foam (161 mg, 64% over two steps). **¹H NMR** (400 MHz, CDCl₃) δ 7.95 (d, *J* = 7.2 Hz, 2H), 7.85 (dd, *J* = 13.4, 7.3 Hz, 6H), 7.73 - 7.17 (m, 49H), 5.54 - 5.39 (m, 2H), 5.33 - 5.21 (m, 2H), 5.14 - 5.05 (m, 3H), 4.96 (t, *J =* 8.5 Hz, 1 H), 4.80 - 4.69 (m, 2H), 4.58 (d, *J =* 12.3 Hz, 1 H), 4.49 (dd, *J* = 7.6, 5.9 Hz, 2H), 4.40 - 4.29 (m, 5H), 4.28 - 4.20 (m, 1H), 4.16 (d, *J* = 11.7 Hz, 1 H), 4.01 (dt, *J* = 27.9, 9.2 Hz, 3H), 3.93 - 3.83 (m, 1 H), 3.76 - 3.61 (m, 1 H), 3.60 - 3.51 (m, 2H), 3.48 (t, *J* = 7.9 Hz, 2H), 3.46 - 3.37 (m, 3H), 3.34 (dd, *J* = 15.4, 6.8 Hz, 3H), 3.28 - 3.12 (m, 4H), 3.07 - 2.94 (m, 4H), 2.94 - 2.87 (m, 2H), 1.37 - 1.21 (m, 4H), 1.14 - 0.98 (m, 2H). **¹³C NMR** (101 MHz, CDCl₃) δ 165.1 (2C), 165.0, 164.9, 164.6, 163.7, 137.93, 137.2, 135.2, 133.4, 133.0, 133.0, 132.9, 132.8, 132.5, 130.0, 129.6, 129.59, 129.5, 129.4, 129.3, 128.8, 128.7, 128.5, 128.4, 128.3, 128.2, 128.2, 128.1, 128.0, 127.9, 127.9, 127.8, 127.6, 127.2, 126.6, 126.1, 125.9, 125.6, 100.9 (2C), 100.1, 84.9, 82.0, 77.2, 75.7, 75.5, 75.3, 74.8, 74.50, 74.5, 74.1, 73.6, 73.4, 73.2, 72.0, 71.7, 71.5, 70.2, 69.2, 67.0, 62.3, 61.6, 50.5, 47.0, 46.0, 28.9, 28.8, 22.9. **HRMS** (ESI⁺) calculated for C₁₁₁H₁₀₉NO₂₉Na [M + Na]⁺ 1942.6983, found 1942.7086.

### Example C.4 Synthesis of tetrasaccharide

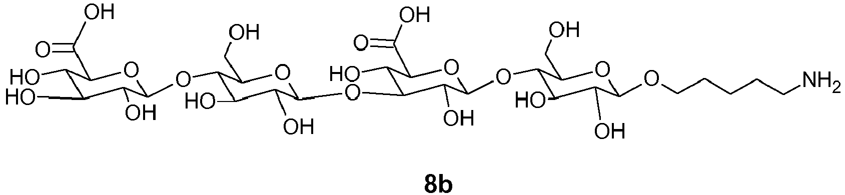

a) Tetrasaccharide **4b** (160 mg, 0.083 mmol) was taken in a mixture of dichloromethane (2.5 mL) and water (0.5 mL) and cooled to 0 °C. TEMPO (2.6 mg, 0.017 mmol) and BAIB (188 mg, 0.583 mmol) were added and the mixture was stirred at 0 °C for 20 min and slowly warmed to room temperature, followed by additional stirring for 5 h. The reaction mixture was then diluted with dichloromethane (10 mL) and water (5 mL) and the aqueous layer was extracted with dichloromethane (4 x 10 mL). The combined organic extracts were dried over Mg₂SO₄ and concentrated *in vacuo.* The crude product was then purified by flash chromatography using dichloromethane (100%), dichloromethane/acetone (10:1) and finally dichloromethane/acetone/AcOH (100:10:1) to obtain 5-(benzyl(benzyloxycarbonyl)amino)pentyl 2-*O*-benzoyl-3-*O*-(2-naphthylmethyl)-β-D-glucopyranuronosyl-(1→4)-2,3-di-*O*-benzoyl-6-*O*-benzyl-β-D-glucopyranosyl-(1→3)-2-*O*-benzoyl-β-D-glucopyranuronosyl-(1→4)-2,3-di-*O*-benzoyl-6-*O*-benzyl-β-D-glucopyranoside **5b** as an off-white solid. This diacid **5b** was transferred to the next reaction without additional purification, assuming total conversion to the desired product (100% yield).
b) Tetrasaccharide **5b** (143 mg, 0.073 mmol) was taken in 0.5 M methanolic solution of sodium methoxide (20 mL, 1.07 mmol) and dichloromethane (1-4 mL) and the resulting solution was stirred at 50 °C for 4 days. The reaction mixture was evaporated in vacuum and then diluted with methanol (5 mL) and neutralized by addition of Amberlite^{®} IR120 H⁺ resin. The clear suspension was filtered and washed thoroughly with methanol. The filtrate was concentrated in vacuum. The remaining colorless solid 5-(benzyl(benzyloxycarbonyl)amino)-pentyl 3-*O*-(2-naphthylmethyl)-β-D-glucopyranuronosyl-(1→4)-6-*O*-benzyl-β-D-gluco-pyranosyl-(1→3)-β-D-glucopyranuronosyl-(1→4)-6-*O*-benzyl-β-D-glucopyranoside **7b** was transferred to the next step without additional purification.
c) Tetrasaccharide **7b** (97 mg, 0.073 mmol) was taken in a mixture of H₂O (2 mL), *^{t}*BuOH (4 mL) and dichloromethane (0.3 mL), and added to 20% Pd/C (97 mg). The solution was degassed under reduced pressure and subsequently stirred over a hydrogenous atmosphere for 24 h. The reaction mixture was then filtered through a PTFE hydrophobic filter and the filter washed thoroughly with water and methanol. After evaporation of the filtrate and drying under vacuum, target compound 5-aminopentyl β-D-glucopyranuronosyl-(1→4)-β-D-glucopyranosyl-(1→3)-β-D-glucopyranuronosyl-(1→4)-β-D-glucopyranoside **8b** was obtained as an off-white solid (35 mg, 61 % over three steps). **¹H NMR** (600 MHz, D₂O) δ 4.49 - 4.30 (m, 3H), 3.98 (dd, *J* = 12.5, 9.9 Hz, 1 H), 3.88 - 3.76 (m, 5H), 3.73 - 3.64 (m, 6H), 3.63 - 3.33 (m, 16H), 3.29 - 3.21 (m, 3H), 3.16 (t, *J* = 7.4 Hz, 1 H), 2.88 (t, *J =* 7.5 Hz, 2H), 1.61 - 1.50 (m, 4H), 1.38 - 1.29 (m, 2H). **HRMS** (ESI⁻) calculated for C₂₉H₄₈NO₂₉ [M - H]⁻ 778.2617, found 778.2672.

## Claims

1. A synthetic method comprising the following steps:
a) providing a disaccharide of formula **2** 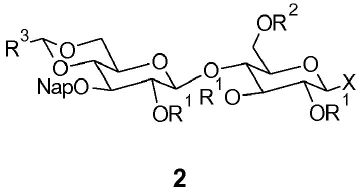 wherein
R¹ represents an oxidation-stable and acid-stable protecting group,
R² represents an oxidation-stable and acid-stable protecting group,
R³ is an aryl or alkyl group, and
X is a leaving group;
and a disaccharide of formula **3** 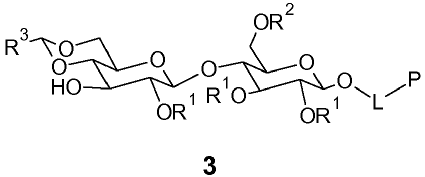
wherein
R¹ represents an oxidation-stable and acid-stable protecting group,
R² represents an oxidation-stable and acid-stable protecting group,
R³ is an aryl or alkyl group,
P is -NR'R" or -N₃,
R and R represent independently of each other -Bn, -Cbz, -Allyl or
R and R form together phthaloyl,
L represents -(CH₂)ₙ-,
n is an integer selected from 2, 3, 4, 5, 6, 7, 8, 9 and 10;
b) coupling of the disaccharide of formula **2** with the disaccharide of formula **3** in presence of a catalyst to obtain a tetrasaccharide of formula **1** 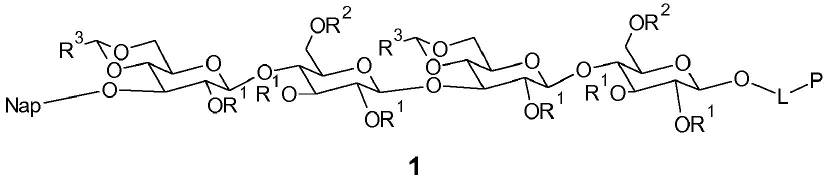 wherein
R¹ represents an oxidation-stable and acid-stable protecting group,
R² represents an oxidation-stable and acid-stable protecting group,
R³ is an aryl or alkyl group,
P is -NR'R" or -N₃,
R and R represent independently of each other -Bn, -Cbz, -Allyl or
R and R form together phthaloyl,
L represents -(CH₂)ₙ-,
n is an integer selected from 2, 3, 4, 5, 6, 7, 8, 9 and 10.

2. The method according to claim 1, further comprising performing selective removal of the acetal groups by treatment of the tetrasaccharide of formula **1** according to claim 1 with a nucleophile in presence of a catalytic amount of an acid to yield a tetrasaccharide of formula **4,** 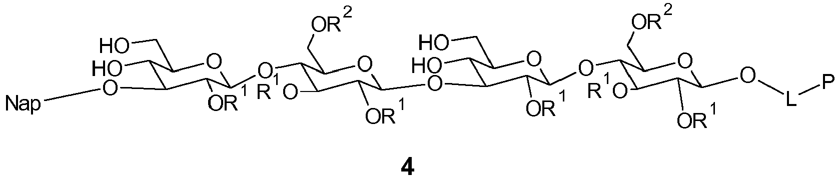 wherein
R¹ represents an oxidation-stable and acid-stable protecting group,
R² represents an oxidation-stable and acid-stable protecting group,
P is -NR'R" or -N₃,
R and R represent independently of each other -Bn, -Cbz, -Allyl or
R and R form together phthaloyl,
L represents -(CH₂)ₙ-,
n is an integer selected from 2, 3, 4, 5, 6, 7, 8, 9 and 10.

3. The method according to claim 2, further comprising performing oxidation of the tetrasaccharide of formula **4** according to claim 2 to yield a tetrasaccharide of formula **5,** 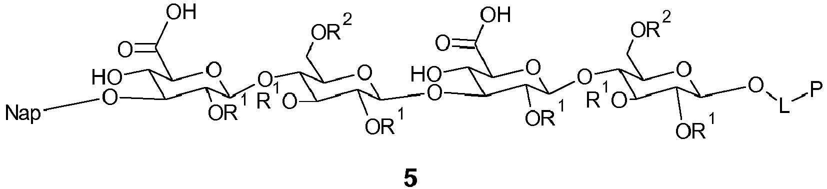 wherein
R¹ represents an oxidation-stable and acid-stable protecting group,
R² represents an oxidation-stable and acid-stable protecting group,
P is -NR'R" or -N₃,
R and R represent independently of each other -Bn, -Cbz, -Allyl or
R and R form together phthaloyl,
L represents -(CH₂)ₙ-,
n is an integer selected from 2, 3, 4, 5, 6, 7, 8, 9 and 10.

4. The method according to claim 3, further comprising performing removal of protecting groups R¹ of tetrasaccharide of formula **5** according to claim 3 in presence of a base to yield a tetrasaccharide of formula **7,** 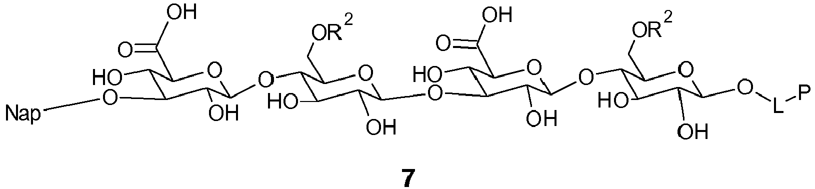 wherein
R¹ represents an oxidation-stable and acid-stable protecting group,
R² represents an oxidation-stable and acid-stable protecting group,
P is -NR'R" or -N₃,
R and R represent independently of each other -Bn, -Cbz, -Allyl or
R and R form together phthaloyl,
L represents -(CH₂)ₙ-,
n is an integer selected from 2, 3, 4, 5, 6, 7, 8, 9 and 10.

5. The method according to claim 4, further comprising hydrogenating the tetrasaccharide of formula **7** according to claim 4 in presence of a catalyst to yield a tetrasaccharide of formula **8,** 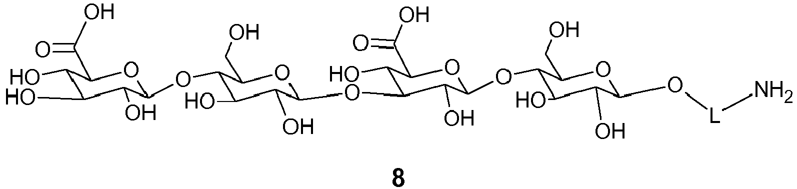
wherein L represents -(CH₂)ₙ- and
n is an integer selected from 2, 3, 4, 5, 6, 7, 8, 9 and 10.

6. The method according to any one of claims 1 - 5 further comprising a6) reacting the disaccharide of formula **2** according to claim 1 with an alcohol HO-L-P in presence of a catalyst to yield a disaccharide of formula **9** 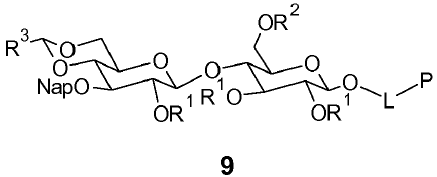 wherein
R¹ represents an oxidation-stable and acid-stable protecting group,
R² represents an oxidation-stable and acid-stable protecting group,
R³ is an aryl or alkyl group,
P is -NR'R" or -N₃,
R and R represent independently of each other -Bn, -Cbz, -Allyl or
R and R form together phthaloyl,
L represents -(CH₂)ₙ-,
n is an integer selected from 2, 3, 4, 5, 6, 7, 8, 9 and 10.

7. The method according to claim 6, further comprising b6) converting the disaccharide of formula **9** according to claim 6 to the disaccharide of formula **3** according to claim 1 by treatment with an oxidizing agent.

8. The method according to any one of claims 1 - 7 further comprising:
a2) providing a monosaccharide building block of formula **10** 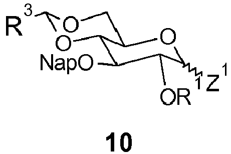 wherein
R¹ represents an oxidation-stable and acid-stable protecting group,
R³ is an aryl or alkyl group,
and Z¹ is a leaving group,
and a monosaccharide building block **11** 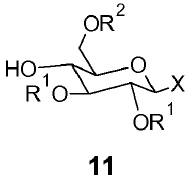
wherein
R¹ represents an oxidation-stable and acid-stable protecting group,
R² represents an oxidation-stable and acid-stable protecting group, and
X is a leaving group;
and
b2) coupling of the monosaccharide building block of formula **10** with the monosaccharide building block of formula **11** in presence of a catalyst to yield the disaccharide of formula **2** according to claim 1.

9. The method according to claim 8, further comprising:
a3) providing a monosaccharide of formula **14** 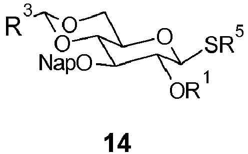 wherein
R¹ is an oxidation-stable and acid-stable protecting group,
R³ is an aryl or alkyl group, and
R⁵ is selected from -Me, -Et, -Pr, -Bu, -Ph, and -Tol;
and
b3) converting the monosaccharide of formula **14** to a monosaccharide of formula **13** 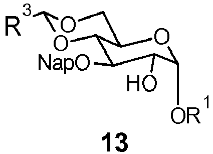 wherein
R¹ is an oxidation-stable and acid-stable protecting group,
R³ is an aryl or alkyl group;
and
c3) treatment of the monosaccharide of formula **13** with a base to obtain a monosaccharide of formula **12** 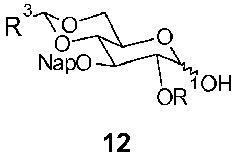 wherein
R¹ is an oxidation-stable and acid-stable protecting group,
R³ is an aryl or alkyl group;
and
d3) converting the monosaccharide of formula **12** to the monosaccharide of formula **10** according to claim 8.

10. The method according to any one of claims 1 - 9, wherein
R¹ is selected from -Bz, -ClAc, -Ac and -Piv,
R² represents -Bn
R³ is selected from -Ph, -PMP and -Me,
P is -NR'R",
R and R represent independently of each other -Bn, -Cbz, -Allyl, and
n is an integer selected from 2, 3, 4 and 5.

11. The method according to any one of claims 1 - 10, wherein
X represents -SR⁵, with R⁵ being selected from -Me, -Et, -Pr, -Bu, -Ph and -Tol and/or Z¹ being selected from -O-C(=NH)-CCl₃, -O-C(=NPh)-CF₃, -OAc or -SR⁵, with R⁵ being selected from -Me, -Et, -Pr, -Bu, -Ph and -Tol.

12. Intermediates of the following general formula: 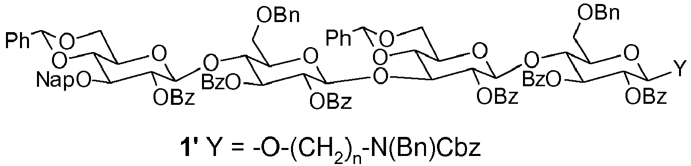 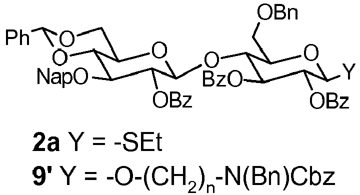 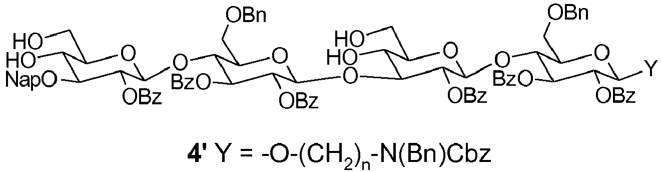 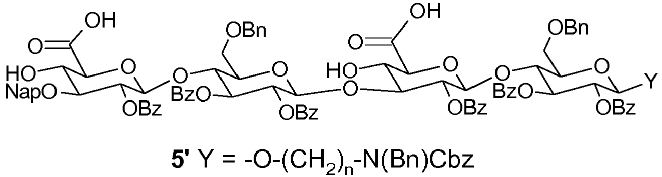 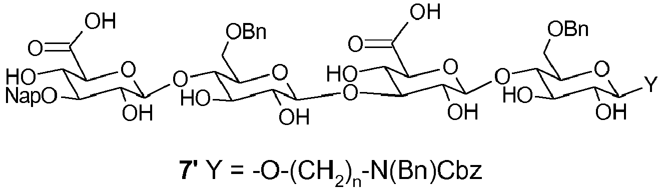 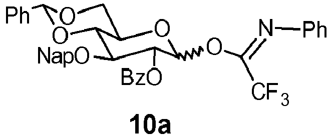 wherein n is an integer selected from 2, 3, 4, 5, 6, 7, 8, 9 and 10.

13. Intermediates according to claim 12, wherein intermediates **1'**, **2a, 4', 5', 7', 9'** and **10a** are crystalline.

14. The method according to claim 5 further comprising:
a4) reacting the tetrasaccharide of formula **8** with a linker J¹-L²-J² under addition of a base to yield tetrasaccharide of formula **16,** 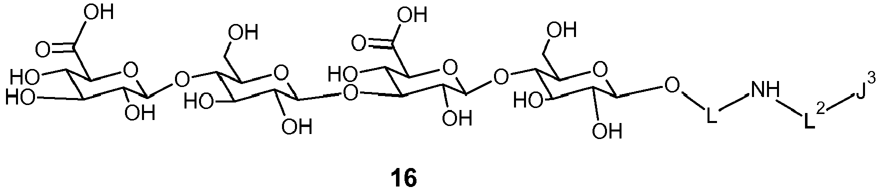
wherein L represents -(CH₂)ₙ-,
n is an integer selected from 2, 3, 4, 5, 6, 7, 8, 9 and 10,
J³ is J¹ or J²,
L² is selected from: 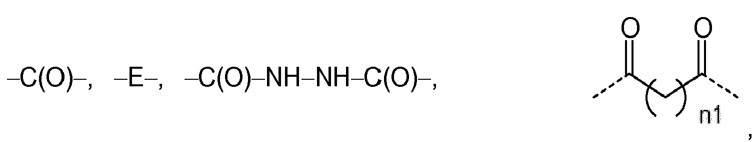 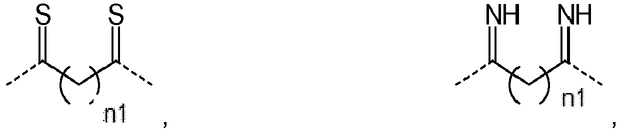 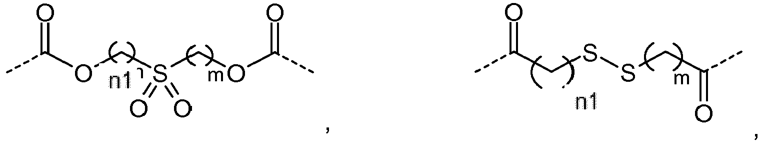 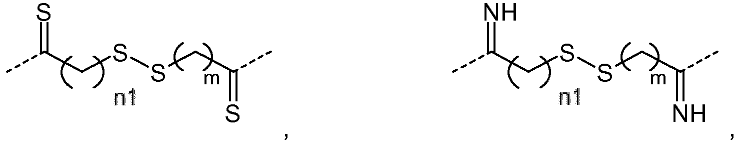 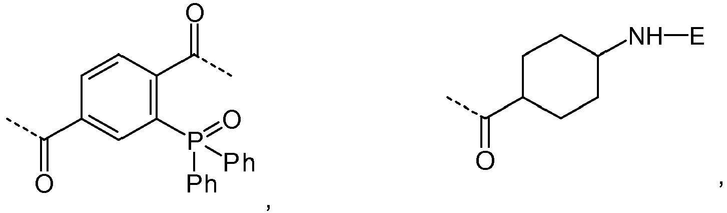 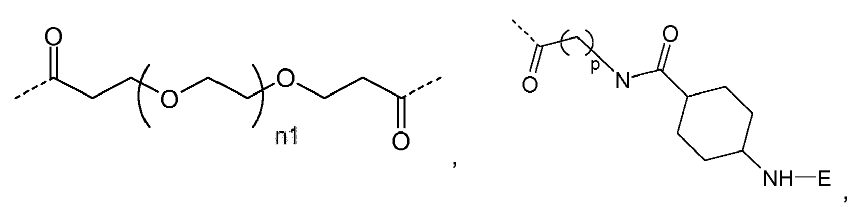 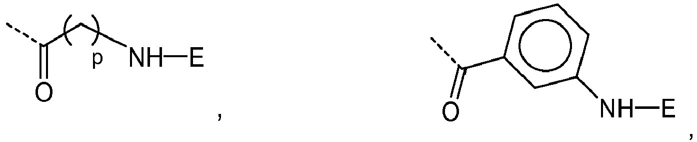 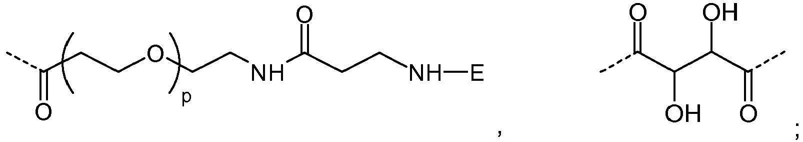
E is selected from 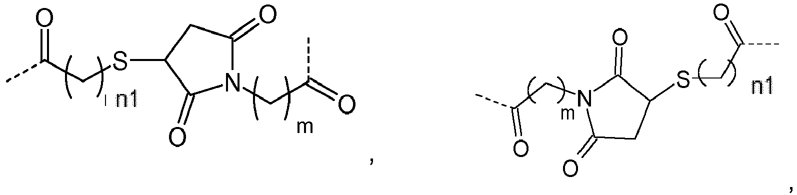 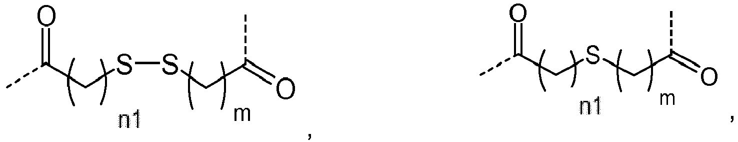 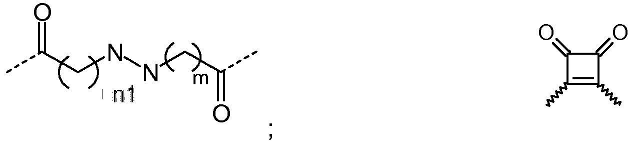
m, n1, o and p represent independently of each other an integer from 1 to 10 and
J¹ and J² are selected independently of each other from: 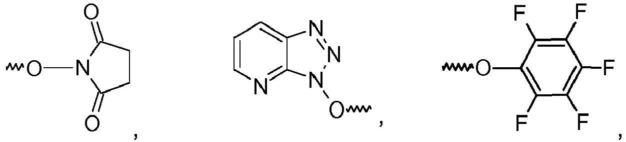 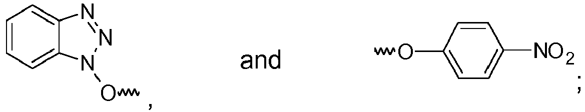 and
b4) coupling the tetrasaccharide of formula **16** to a carrier protein.
